(19) Europäisches Patentamt
European Patent Office
Office européen des brevets

(11) Publication number: **0 454 146 A1**

(12) **EUROPEAN PATENT APPLICATION**

(21) Application number: **91106789.0**

(22) Date of filing: **26.04.91**

(51) Int. Cl.5: **C07C 49/733**, C07F 15/06, C07C 321/20, C07F 7/18, A61K 31/12

(30) Priority: **30.11.90 US 621503**
**27.04.90 US 515387**

(43) Date of publication of application:
**30.10.91 Bulletin 91/44**

(84) Designated Contracting States:
**AT BE CH DE DK ES FR GB GR IT LI LU NL SE**

(71) Applicant: **Bristol-Myers Squibb Company**
**345 Park Avenue**
**New York, N.Y. 10154(US)**

(72) Inventor: **Kadow, John F.**
**126 Metacomet Drive**
**Meriden, Connecticut 06450(US)**

(74) Representative: **Kinzebach, Werner, Dr. et al**
**Patentanwälte Reitstötter, Kinzebach und Partner Sternwartstrasse 4 Postfach 86 06 49**
**W-8000 München 86(DE)**

(54) **Cytotoxic bicyclo[7.3.1] tridec-4-ene-2,6-diyne compounds and process for the preparation thereof.**

(57) The present invention relates to a novel and efficient process for the preparation of 8-hydroxy-bicyclo[7.3.1]-tridec-4-ene-2,6-diyne ring system which is part of the aglycone of esperemicin and to novel cytotoxic agents having said bicyclic ring system. The present invention also provides a method for treating animal malignant tumors by administering to an animal in need of such treatment an antitumor effective amount of a compound having the formula

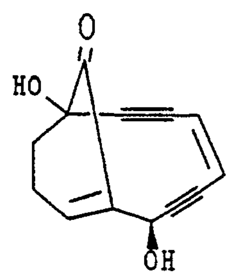

EP 0 454 146 A1

## BACKGROUND OF THE INVENTION

1. Field of the Invention

The present invention relates to cytotoxic DNA-cleaving compounds, a novel process for their preparation, and intermediates produced thereby.

2. Background Art

Esperamicins and calichemicins belong to a class of extremely potent antitumor antibiotics isolated from microbial sources. Structure elucidation studies of the esperamicins and calichemicins were reported in J. Am. Chem. Soc., 1987, 109:3461-3462, and J. Am. Chem. Soc., 1987, 109:3464-3466, respectively. These antibiotics share a common aglycone core which contains a bicyclo[7.3.1]tridecane ring system with an allylic trisulfide side chain.

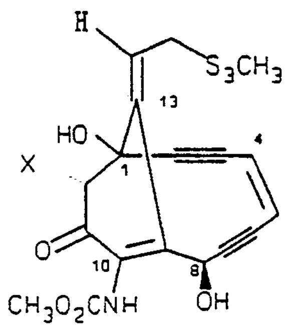

calichemicin aglycone: X = H
esperamicin aglycone: X = OH

The proposed mechanism of action of these antibiotics involves, first, a bioreductive activation of the trisulfide to generate a thiol which adds intramolecularly to the a,$\beta$-unsaturated enone. The resulting change of hybridization of the bridgehead carbon atom brings the two ends of the diynene portion into closer proximity to coalesce and form a benzene 1,4-diradical which is capable of abstracting a hydrogen atom from the sugar phosphate backbone of DNA to effect single and double stranded breakage.

The unique structure and mechanism of action of these compounds have engendered much interest in the synthesis of the bicyclic diynene core fragment. A number of strategies have been devised to achieve ring closure of a cyclohexyl compound bearing the requisite diynene fragment to form the 10-membered ring.

Kende, et al., (Tet. Lett., 1988, 29:4217-4220) treated 3,3-(1,2-ethylenedioxy)-5-(3-hexene-1,5-diynyl)-1-cyclohexenecarboxaldehyde with lithium bis(trimethylsilyl)amide, followed by removal of the ethylenedioxy ketone protecting group to provide 8-hydroxy-bicyclo[7.3.1]tridec-4,9-diene-2,6-diyn-11-one.

Magnus, et al., (J. Am. Chem. Soc., 1988, 110:1626-1628) reported the preparation of 1-(TBSoxy)-bicyclo[7.3.1]tridec-4-ene-2,6-diyn-10-one, dicobalt hexacarbonyl complex [TBS = t-butyldimethylsilyl] from 1,4-bis(TBSoxy)-4-(7-methoxy-3-heptene-1,5-diynyl)cyclohexene dicobalt hexacarbonyl complex upon treatment with titanium tetrachloride/diazabicyclo-[2.2.2]octane (DABCO) at -78°C. Decomplexation of the product, however, caused the molecule to collapse into the corresponding benzenoid compound.

Magnus, et al., (J. Am. Chem. Soc., 1988, 110:6921-6923) and Tomioka, et al., (Tet. Lett., 1989, 30:851-854) reported the preparation of 1-(TBSoxy)bicyclo[7.3.1]tridec-4-ene-2,6-diyn-13-one (bicyclic ketone) from 1,6-bis-(TBSoxy)-6-(7-methoxy-3-heptene-1,5-diynyl)cyclohexene dicobalt hexacarbonyl complex upon treatment with titanium tetrachloride/DABCO, followed by decomplexation with iodine or trimethylamine oxide. Magnus, et al., further treated the bicyclic ketone product with potassium hexamethyldisilazene (KHMDS) and phenylselenium chloride to form the $\alpha$-phenylselenide which, upon oxidation with hydrogen peroxide, provided 1-(TBSoxy)-bicyclo[7.3.1]tridec-4,9-diene-2,6-diyn-13-one (bicyclic enone). This latter product was also obtained as a minor product when the TBS enol ether of the bicyclic ketone was oxidized with selenium dioxide (Magnus, et al., Tet. Lett., 1989, 30:3637-3640).

Danishefsky, et al., (J. Am. Chem. Soc., 1988, 110:6890-6891) reported the preparation of 1-(TBSoxy)-8-hydroxy-11-methoxy-bicyclo[7.3.1]tridec-4,9,11-triene-2,6-diyne 13-spiroethylene epoxide from 3-methoxy-5-(TBSoxy)-5-(3-hexene-1,5-diynyl)-1,6-cyclohexadienecarboxaldehyde 6-spiro ethylene epoxide upon treatment with base. The product was further elaborated to provide inter alia 1,8-dihydroxy-bicyclo[7.3.1]tridec-4,9-diene-2,6-diyn-11,13-dione and the corresponding 11-ethylene ketal, and 1,8-dihydroxy-bicyclo[7.3.1]-

tridec-4-ene-2,6-diyn-11,13-dione. This latter compound was shown to cleave DNA in vitro (J. Org. Chem., 1989, 54:2781-2783 and J. Am. Chem. Soc., 1989, 111:7638-7641).

Magnus, et al., (J. Org. Chem., 1990, 55(6):1709-1711) reported the preparation of 8-hydroxy-1-TBSoxy-bicyclo[7.3.1]tridec-4-ene-2,6-diyn-13-one by treating 6-TBSoxy-6-(7-oxo-3-hexene-1,5-diynyl)-cyclohexanone dicobalt complex with dibutylboron triflate/DABCO to effect ring closure, followed by N-methyl-morpholine oxide to remove the cobalt carbonyl group.

Danishefsky, et al., (J. Am. Chem. Soc., 1990, 112:3253-3255) reported the total synthesis of dl-calicheamicinone.

The known methods for ring closure either require the use of cumbersome precursors which are difficult to prepare, or they yield bicyclic diynenes lacking certain key functionalities. The process of the present invention circumvents these problems and provides a highly efficient route to bicyclic diynenes with multiple key functionalities. Furthermore, the present process results in the formation of a single pair of diastereomers and allows the introduction of the 8-hydroxy group having the same relative stereochemical configuration as the 8-hydroxy group of the esperamicin aglycone.

## SUMMARY OF THE INVENTION

The present invention provides, in one aspect, a process for closing a 10-membered ring to form an 8-hydroxy-bicyclo[7.3.1]tridec-4-ene-2,6-diyne-13-one ring system. The process comprises the steps of: 1) reacting a dicobalt hexacarbonyl complexed 6-protected hydroxy 6-(7-oxo-3-heptene-1,5-diynyl)-2-cyclohexenone derivative with a nucleophilic species (Nu) capable of 1,4-conjugated addition to the enone; and 2) treating the resultant reaction product with a titanium reagent to effect ring closure to form the corresponding dicobalt hexacarbonyl complexed 1-protected hydroxy 10-Nu-8-hydroxy-bicyclo[7.3.1]tridec-4-ene-2,6-diyne-13-one derivative. The decomplexation of the compound obtained by this process is also part of the present invention.

Another aspect of the present invention provides a process for preparing an 8-hydroxy-bicyclo[7.3.1]-tridec-4,9-diene-2,6-diyne-13-one compound which comprises oxidative elimination of the 10-Nu substituent of 10-Nu-8-hydroxy-bicyclo[7.3.1]tridec-4-ene-2,6-diyne-13-one.

Another aspect of the present invention provides 10-Nu-8-hydroxy-bicyclo[7.3.1]tridec-4-ene-2,6-diyne-13-one derivatives, and their cobalt complexes.

Also provided by the present invention are certain 8-hydroxy-bicyclo[7.3.1]tridec-4,9-diene-2,6-diyne-13-one derivatives.

## DETAILED DESCRIPTION OF THE INVENTION

The numbering of the bicyclo[7.3.1]tridec-4-ene-2,6-diyne ring system referred to in the specification is as follows:

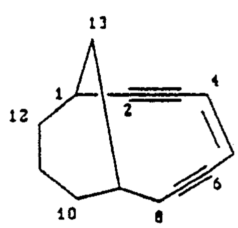

"10-Membered ring" is the ring defined by carbon atoms 1-9 and 13 of the bicyclo[3.7.1]tridec-4-ene-2,6-diyne ring system.

Dicobalt hexacarbonyl complexed carbon-carbon triple bond is represented by

$|||\ Co_2(CO)_6$ .

This group is also referred to in the specification as "cobalt carbonyl complex" or "cobalt complex". The dicobalt hexacarbonyl group may be used as carbon-carbon triple bond protecting group. Cobalt complexed acetylene is the subject of the review by Nicholas, K. M., Accounts in Chemical Research, 1987, 20:207-214.

"TBS" is used throughout the specification as an abbreviation for t-butyldimethylsilyl [also referred to as

(1,1-dimethylethyl)dimethylsilyl].

Novel Process

The present invention provides a process for closing a 10-membered ring to form an 8-hydroxy-bicyclo-[7.3.1]tridec-4-ene-2,6-diyne-13-one derivative which comprises the steps of: 1) reacting a dicobalt hexacarbonyl complexed 6-protected hydroxy 6-(7-oxo-3-heptene-1,5-diynyl)-2-cyclohexenone of formula I with a nucleophilic species (Nu) capable of 1,4-conjugated addition to the enone; and 2) treating the resultant reaction product with a titanium reagent to provide the corresponding dicobalt hexacarbonyl complexed 1-protected hydroxy 10-Nu-8-hydroxy-bicyclo[7.3.1]tridec-4-ene-2,6-diyne-13-one of formula III. The nucleophilic species is generally an organometallic reagent Nu-M wherein Nu is a nucleophilie capable of 1,4-conjugated addition to an $\alpha,\beta$-unsaturated carbonyl compound, and M is a monovalent metal cation or a substituted metal having valency higher than one. This process is illustrated in Scheme I in which preferred reagents are exemplified. It will be appreciated that although preferred reagents are used to illustrate the invention in the following Schemes, the invention is by no means limited thereto.

Scheme I

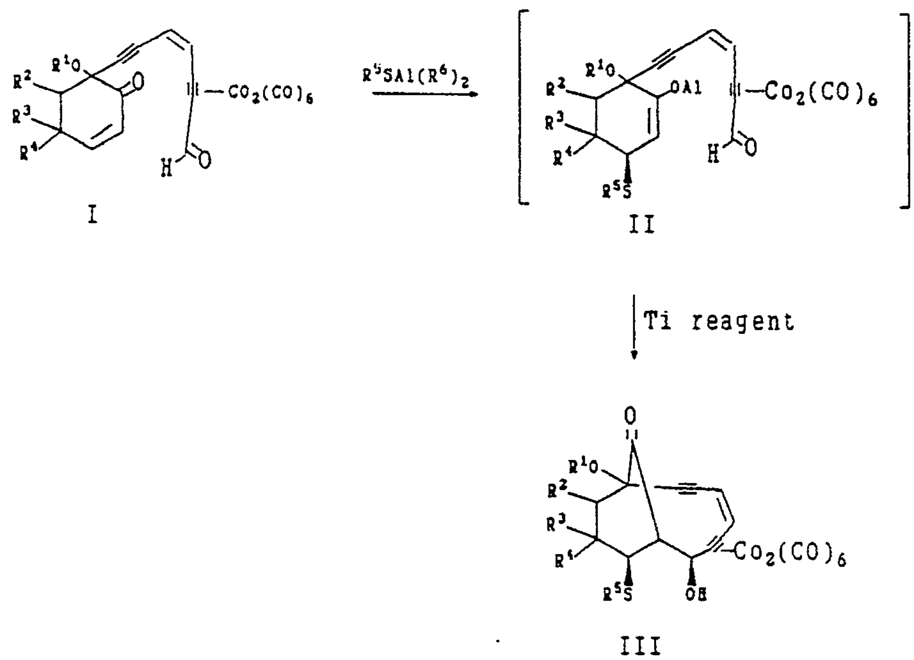


I II III

In the above Scheme, $R^1$ is a hydroxy protecting group; $R^2$ is hydrogen or a protected hydroxy group; $R^3$ and $R^4$ are independently hydrogen or protected hydroxy group; or $R^3$ and $R^4$ taken together with the carbon atom to which they are attached represent a protected keto group. $R^5SA1(R^6)_2$ exemplifies Nu-M; $R^5$ and $R^6$ are selected from is $C_{1-5}$ alkyl, $C_{3-6}$ cycloalkyl, $C_{6-10}$ aryl, or $C_{6-10}$ aryl substituted with one or more groups selected from $C_{1-5}$ alkyl and $C_{1-5}$alkoxy.

The choice of hydroxy protecting group and ketone protecting groups is not particularly limited; the protecting groups may be any that can be readily replaced with hydrogen under conditions which do not affect other functional groups in the molecule.

Examples of hydroxy protecting group include, but are not limited to, a) formation of ether linkage with i) lower alkyl or lower alkenyl group, e.g., methyl, ethyl, propyl, isopropyl, butyl, t-butyl, pentyl, and propenyl; ii) aralkyl group, e.g., benzyl, diphenylmethyl, triphenylmethyl, and tris(p-methoxyphenyl)methyl; and iii) triorganosilyl group, e.g., trimethylsilyl, t-butyldimethylsilyl, t-butyldiphenyl-silyl, triisopropylsilyl; b) formation of acetal or ketal with, for example, tetrahydropyran, methoxymethyl, methoxyethoxymethyl, methylthiomethyl, tetrahydrothiofuranyl, and tetrahydrothiopyranyl; and c) formation of ester with i) optionally substituted lower alkanoyl, e.g., formyl, acetyl, propionyl, butyryl, trifluoroacetyl, chloroacetyl, methoxyacetyl, and phenoxyacetyl; ii) benzoyl or p-nitrobenzoyl; and iii) optionally substituted alkoxycarbonyl, e.g., methoxycarbonyl, ethoxycarbonyl, t-butyloxycarbonyl, isobutyloxycarbonyl, trichloroethoxycarbonyl, and

tribromoethoxycarbonyl. Preferred hydroxy protecting group is t-butyldimethylsilyl.

A ketone protecting group may be one in which the oxo functionality has been converted into a ketal group. Examples of suitable ketal groups include, but are not limited to, a) dialkyl ketals such as dimethyl and diethyl ketals, and 2,2,2-trichloroethyl ketal; b) cyclic ketals such as 1,3-dioxolan, 1,3-dioxan, 2,2-dimethyl-1,3-dioxan, and 4-bromomethyl-1,3-dioxolan; c) thio ketals and hemithioketals such as 1,3-oxathiolan, 1,3-oxathian, 1,3-dithian, 1,3-dithiolan, and 2,2-di(lower alkyl)-1,3-dithian. Preferred ketone protecting groups are cyclic ketals such as 1,3-dioxolan and 1,3-dioxan.

In one preferred embodiment, $R^2$ is hydrogen. In another preferred embodiment, $R^3$ and $R^4$ are both hydrogen. Yet in another preferred embodiment, $R^1$ is a triorganosilyl group. In a more preferred embodiment, $R^2$, $R^3$, and $R^4$ are each hydrogen, and $R^1$ is a triorganosilyl group; most preferably, $R^1$ is t-butyldimethylsilyl.

According to Scheme I, a compound of formula I is treated with a thioaluminum compound having the formula $R^5S\text{-}Al(R^6)_2$, wherein $R^5$ and $R^6$ are organic residues such as $C_{1-5}$alkyl, $C_{3-6}$cycloalkyl, $C_{6-10}$aryl, and $C_{6-10}$aryl substituted with one or more groups selected from alkyl and alkoxy. Preferred thioaluminum compounds are dialkyl(phenylthio)aluminum, for example, dimethyl(phenylthio)aluminum, i.e., $(CH_3)_2AlSC_6H_5$. It will be understood that the thioaluminum reagent is an example of an organometallic compound, Nu-M as previously defined. In general, for Nu-M, the metal cation M may be one commonly used to stabilize an enolate, for example, an alkali metal, alkaline earth metal, aluminum, zinc, and the like; preferably, the metal cation is aluminum. The nucleophilic species Nu is one capable of 1,4-conjugated addition to an $\alpha,\beta$-unsaturated carbonyl compound, and which may be eliminated upon oxidation to regenerate a carbon-carbon double bond; examples of such nucleophilic species include sulfur and selenium nucleophiles; preferably, the nucleophilic species is a sulfur nucleophile.

The reaction is carried out in an inert aprotic organic solvent such as tetrahydrofuran, hexane, etc., or a mixture thereof, at temperature of below ambient temperature, suitably at $0^\circ C$ or below. The reaction generates <u>in situ</u> an aluminum enolate of formula II which may be directly subjected to the ring closure reaction described infra. Alternatively, the enolate may be converted to the corresponding ketone using methods well known in the art, and the ketone may then be used in the subsequent ring closure. Although either the enolate or the ketone may be used in the ring closure step, the enolate is the preferred reactant for the present process.

The reaction mixture containing the aluminum enolate of formula II is treated with a titanium reagent to effect intramolecular ring closure to give novel cobalt complexed bicyclic diynene of formula III. Examples of suitable titanium reagents include, but are not limited to, titanium alkoxides $Ti[O\text{-}(C_{1-5})alkyl]_4$ and titanium alkoxide halides $XTi[(O\text{-}(C_{1-5})alkyl]_3$ wherein X is halogen, such as bromine, chlorine, and iodine. Some specific examples of titanium reagents that may be mentioned are titanium isopropoxide $Ti(OCH(CH_3)_2)_4$, titanium propoxide $Ti(OCH_2CH_2CH_3)_4$, titanium ethoxide $Ti(OCH_2CH_3)_4$, and titanium isopropoxide chloride $Ti(OCH(CH_3)_2)_3Cl$. The nucleophile $R^5\text{-}S\text{-}Al(R^6)_2$ and the titanium reagent are used in at least equimolar amount, but preferably in excess, relative to the diynene. Thus up to about 10 equivalents of the sulfur nucleophile and up to about 40 equivalents of the titanium reagent may be employed. Preferably the sulfur nucleophile is used in a range of about 1.5 to 10 equivalents, and the titanium reagent in a range of about 1.5 to 40 equivalents relative to the diynene.

The cobalt carbonyl group of compounds of formula III may be removed to provide compounds of formula IV by treatment with known decomplexation agents such as iodine, iron (III) nitrate, and a tertiary amine N-oxide, e.g., N-methylmorpholine-N-oxide, trimethylamine-N-oxide, and the like. The preferred reagent is iodine. The reaction is carried out in an inert organic solvent such as benzene, dichloroethane, etc; a preferred solvent is benzene. The reaction temperature may be any that is conducive to product formation and may be ambient temperature. The decomplexation agent is used at least in equimolar amount relative to the diynene but, preferably, is used in excesss of from about 1.5 to about 10 equivalents.

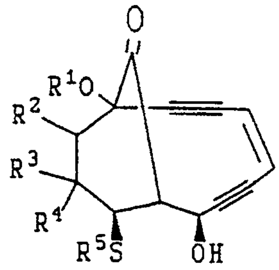

IV

The sulfide substituent at position 10 of the bicyclic diynene of formula IV may be oxidized to the corresponding sulfoxide, the latter group is then eliminated to provide the enone product of formula Va. The dicobalt complexed bicyclic diynene of formula III may be similarly converted to the cobalt complexed enone of formula Vb.

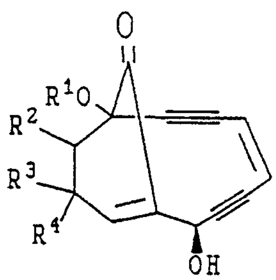

Va

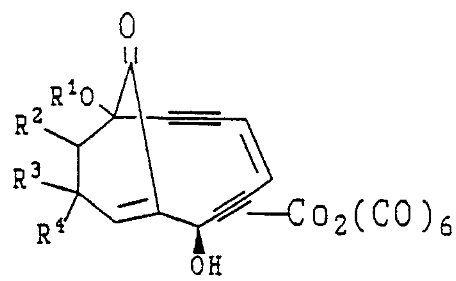

Vb

Methods for oxidation of sulfides and elimination of sulfoxides to provide $\alpha,\beta$-unsaturated carbonyl compounds are generally well known in the art. The sulfide substituent of compounds of formulas IV and III may be oxidized to the corresponding sulfoxide using a variety of reagents, including, but are not limited to, hydrogen peroxide, peracids, periodates, perborates, acyl nitriles, and the like. The reaction is carried out in a suitable inert organic solvent such as lower alcohol or aqueous lower alcohol at a temperature and for a period suitable to cause the elimination of the sulfoxide to form the $\alpha,\beta$-unsaturated bicyclic diynene products of formulas Va and Vb, respectively. Preferably, the reaction is carried out at room temperature, and the reaction is usually complete in less than 10 hours to yield the desired enone product. Compounds of formula Vb may be converted into compounds of formula Va by decomplexation methods earlier described. It will be appreciated that, even though sulfide substituents are exemplified, selenide substitutents may be similarly converted to enones of formulas Va and Vb.

In one preferred embodiment of the invention, the sulfide substituent at position 10 of compounds of formulas IV and III is phenyl sulfide, and the oxidation/elimination reaction is carried out at room temperature using sodium periodate or m-chloroperbenzoic acid (mCPBA) as the oxidizing agent.

Alternatively, a compound of formula III may be converted to the corresponding compound of formula Va in one step by treating the dicobalt hexacarbonyl complexed compound of formula III with meta-chloroperbenzoic acid (mCPBA). The reaction is carried out in an inert organic solvent such as methylene chloride at a temperature conducive to product formation, preferably at about room temperature. The reaction is generally complete in a few hours. mCPBA is used in at least equivalent amount to the bicyclic diynene compound, but preferably, it is used in excess of up to about 4 equivalents of the diynene.

Novel Compounds

Compounds of formulas III and IV are novel compounds prepared by the processes described above. For compounds of formulas III and IV, the $R^1$ hydroxy protecting group may be removed using methods well known in the art to provide the corresponding 1-hydroxy compounds. The deprotection method used depends on the nature of the protecting group and may be, for example, hydrolysis under acidic or basic conditions, hydrogenolysis, and alcoholysis. When $R^1$ is t-butyldimethylsilyl, this group may be removed

with, for example, trifluoromethanesulfonic acid. In compounds of formula III and IV wherein $R^2$ is protected hydroxy, or wherein one of $R^3$ or $R^4$, or both are protected hydroxy, such hydroxy protecting group may also be removed by methods as described above for $R^1$. In compounds of formula III and IV, wherein $R^3$ and $R^4$ together represent a protected ketone, deprotection may be effected by conventional methods such as acid hydrolysis.

Thus, another aspect of the invention provides a bicyclic diynene of formula VIa, as well as its corresponding dicobalt hexacarbonyl complex VIb

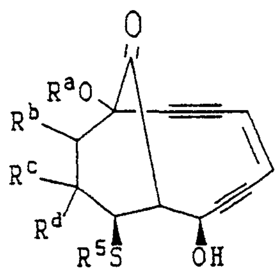

VIa

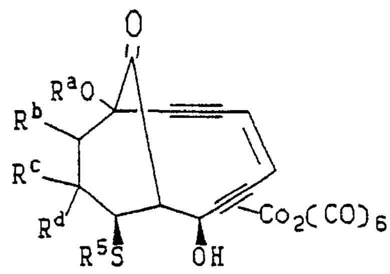

VIb

wherein $R^a$ is hydrogen or a hydroxy protecting group; $R^b$ is hydrogen, hydroxy or a protected hydroxy group, $R^c$ and $R^d$ are independently hydrogen, hydroxy, a protected hydroxy group; or $R^c$ and $R^d$ together are an oxo group or a protected keto group; and $R^5$ is as defined above. A preferred embodiment provides compounds of formulas VIa and VIb wherein $R^a$ is hydrogen or a triorganosilyl group, preferably t-butyldimethylsilyl. Another preferred embodiment provides compounds of formulas VIa and VIb where $R^b$, is hydrogen. Another preferred embodiment provides compounds of formulas VIa and VIb wherein $R^c$ and $R^d$ are each hydrogen. Another preferred embodiment provides compounds of formulas VIa and VIb wherein $R^5$ is as previously defined, and is preferably a $C_{6-10}$aryl or substituted aryl group, and most preferably phenyl or alkoxy substituted phenyl. One particularly preferred embodiment provides compounds of formula VIa and VIb wherein $R^a$ is trialkylsilyl, preferably t-butyldimethylsilyl; $R^b$, $R^c$, and $R^d$ are hydrogen; and $R^5$ is phenyl, or alkyl or alkoxy substituted phenyl.

The present invention also provides novel compounds of formula VII wherein $R^a$ is hydrogen or a hydroxy protecting group; preferably $R^a$ is hydrogen or t-butyldimethylsilyl. A compound of formula VII wherein $R^a$ is a hydroxy protecting group may be converted to the corresponding compound in which $R^a$ is hydrogen by removing the hydroxy protecting group using conventional methods as described hereinabove. In particular, where $R^a$ is t-butyldimethylsilyl deprotection may be accomplished using trifluoromethanesulfonic acid.

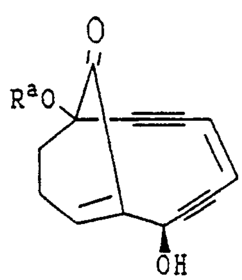

VII

It will be appreciated that the various compounds produced by the novel process of the present invention can exist as optical isomers; the individual isomers, as well as racemic mixtures and diastereomeric mixtures, are all contemplated as being within the scope of the invention. Similarly, the novel process of the invention is applicable to the individual stereoisomers, as well as racemic and diastereomeric mixtures thereof. The stereochemical notations used in the structural formulas depicted in the specification and claims are meant to represent the relative orientations of the various substituents on the bicyclo[7.3.1]-

tridec-4-ene-2,6-diyne ring system and are not meant to restrict the compounds represented by these formulas to specific absolute configurations.

Preparation of Starting Materials

Turning now to the preparation of compounds of formula I which are the starting material used in the novel process of the invention. In the following Schemes, $R^1$, $R^2$, $R^3$, and $R^4$ have the same meanings as defined under formula I.

The cobalt complex diynene aldehyde of formula I may be prepared from the corresponding non-complexed acetal as depicted in Scheme II.

Scheme II

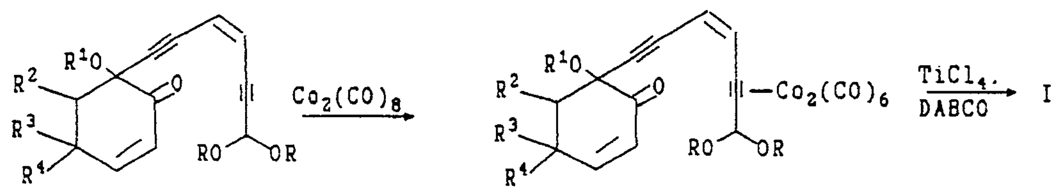


VIII IX

Thus, the diynene acetal VIII is treated with dicobalt octacarbonyl at room temperature in an inert organic solvent such as heptane or methylene chloride. The cobalt complexed diynene acetal IX is converted into the corresponding aldehyde of formula I upon treatment with titanium tetrachloride and 1,4-diazabicyclo[2.2.2]octane (DABCO) at -65° C in methylene chloride. In this Scheme, R is lower alkyl or the two R groups join to form a cyclic acetal. It is to be understood that the order of the two reaction steps depicted in Scheme II may be reversed; i.e., diynene acetal VIII may be first converted to the corresponding aldehyde by acid hydrolysis using, e.g., trifluoroacetic acid/chloroform, followed by complexation with dicobalt octacarbonyl to provide compound of formula I.

The diynene acetal VIII may be constructed from a 2-cyclohexenone derivative and a diynene acetal fragment. Several strategies may be employed to accomplish this end, and these are illustrated in Scheme III.

Scheme III

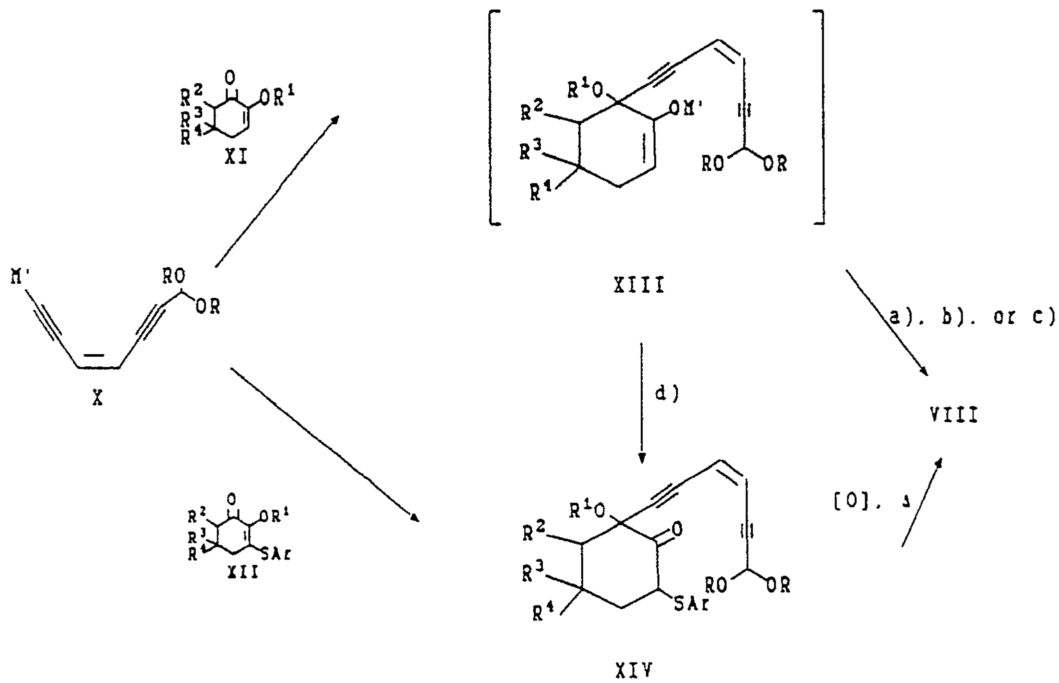


a: 1) $H_2O$; 2) t-butyldimethylsilyltriflate; 3) selenium dioxide
b: 1) t-butyldimethylsilyltriflate; 2) selenium dioxide
c: 1) phenyl selenium chloride; 2) hydrogen peroxide
d: 1) $ArSSO_2Ar$

In Scheme III, M is an alkali metal, for example, lithium, sodium, or potassium. R is a lower alkyl, or the two R groups joined to form a cyclic acetal. Ar is phenyl or phenyl substituted with a lower alkyl or alkoxy such as methyl, ethyl, methoxy, and ethoxy.

To elaborate on Scheme III, the anion of the diynene acetal X is treated with a cyclohexenone of formula XI to generate a diynene-substituted cyclohexanone enolate of formula XIII. Or, the anion X is treated with 3-arylthio substituted 2-cyclohexenone of formula XII to yield a diynene-substituted cyclohexanone of formula XIV. Compounds of formulas XIII and XIV are further elaborated in order to obtain the desired cyclohexenone diynene acetal of formula VIII.

In one process of Scheme III, the anion of the diynene acetal is treated with a 3-arylthio substituted 2-cyclohexenone XII at reduced temperature, e.g., at -78°C. The resultant addition product XIV is then oxidized to sulfoxide using a conventional oxidizing agent, such as mCPBA or sodium periodate, and the reaction is preferably carried out at low temperature, e.g., -78°C. Heating the sulfoxide, e.g., refluxing in carbon tetrachloride or heating in pyridine at about 100°C-110°C generates the cyclohexenone diynene acetal of formula VIII. As a variation of this sequence, the sulfoxide of compound XII may be used in place of XII to yield the sulfoxide of compound XIV. The sulfoxide of XIV is then heated in an organic solvent, such as in refluxing heptyne or pyridine, and optionally in the presence of a reagent for trapping the sulfur elimination product, e.g., 2-mercaptobenzothiazole, to give compound of formula VIII.

In the second process of Scheme III, the anion of the diynene acetal is treated with a cyclohexenone of formula XI in an inert solvent, such as tetrahydrofuran, at low temperature, e.g., from about -78°C to about -50°C to generate the metal enolate XIII which can be trapped and oxidized to the cyclohexenone diynene acetal of formula VIII. In one method, the metal enolate XIII is quenched with water at room temperature, and the resultant ketone is treated with t-butyldimethylsilyltrifluoromethanesulfonate (TBDMS triflate) at room temperature to provide the corresponding silyl enol ether. The silyl enol ether may also be obtained by treating the metal enolate XIII with TBDMS triflate at -78°C. The silyl enol ether is then oxidized using selenium dioxide at elevated temperature, e.g., refluxing temperature of the reaction mixture to provide the enone of formula VIII. Enone of formula VIII can also be prepared by treating the metal enolate XIII with phenylselenium chloride at -78°C, followed by oxidizing the resultant α-phenyl-selenide with hydrogen peroxide. As depicted in Scheme III, the metal enolate XIII may also be treated with $ArSSO_2Ar$ to yield a compound of formula XIV which is converted into a compound of formula VIII as previously described.

The diynene acetal X may be synthesized using the following procedure. Cis-1,2-dichloroethylene is reacted with 3,3-diethoxypropyne in the presence of copper iodide, palladium tetrakis(triphenylphosphine) and n-butylamine, at room temperature and in the absence of light. The product, (Z)-5-chloro-1,1-diethoxy-4-pentene-2-yne is treated with trimethylsilylacetylene in the presence of copper iodide, palladium tetrakis-(triphenylphosphine), and n-butylamine at room temperature and away from light to provide (Z)-7,7-diethoxy-1-trimethylsilyl-3-hepten-1,5-diyne. The corresponding anion is then generated by treatment with an alkali metal hydroxide, such as lithium hydroxide.

Cyclohexenones of formulas XI and XII may be prepared from commercially available starting materials. For example, 2-TBSoxy-2-cyclohexenone (XI, $R^1$ = TBS, $R^2$ = $R^3$ = $R^4$ = H) may be obtained from 1,2-cyclohexanedione upon treatment with a base, such as triethylamine or lithium diisopropylamide (LDA), followed by TBS triflate. 3-(4-Methylphenyl)-2-TBSoxy-2-cyclohexenone (XII, $R^1$=TBS, $R^2$=$R^3$=$R^4$H, Ar= 4-methylphenyl) can be prepared from 1,2-cyclohexanedione upon treatment with a base such as lithium bis(timethylsilyl)amide, followed by 4-methylphenyl 4-methylbenezenethiosulfonate; the resulting product is treated with a base, e.g. triethylamine, and TBS triflate to yield the desired product.

Compounds of formula XI, wherein $R^3$ and $R^4$ are not both H, can be prepared from 1,4-cyclohexanedione. Thus, 1,4-cyclohexanedione is first converted into a mono-protected form (XVa) wherein $R^{3'}$,$R^{4'}$, together with the carbon atom they are attached, represent a protected ketone group.

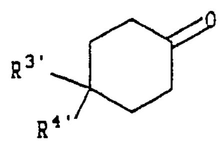

XVa

Preferably, the ketone is protected by conversion to a ketal. Monoketal of formula XV may be obtained upon treatment with a controlled amount of an alcohol, such as methanol, an orthoester, such as methyl orthoformate, a ketal, such as 2,2-dimethoxypropane, or a diol, such as ethylene glycol. Some 1,4-cyclohexanedione monoketals are available commercially, e.g., mono-ethylene ketal and mono-2,2-dimethyltrimethylene ketal.

Mono-protected 1,4-cyclohexanedione (XVa) may be converted to 4-protected hydroxy cyclo hexanone by first reducing the ketone to a hydroxy group using, e.g., sodium borohydride. The hydroxy group may be protected using known methods and reagents, e.g., by treating with a base such as sodium hydride and benzyl bromide. The ketone protecting group is then removed to provide 4-protected hydroxy cyclohexanone

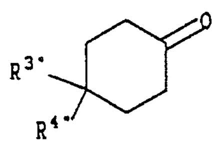

XVb

wherein one of $R^{3''}$ and $R^{4''}$ is hydrogen and the other is a protected hydroxy.

Compounds of formulas XVa and XVb are further elaborated to provide compounds of formula XI, wherein $R^3$ and $R^4$ are not both H. One such reaction sequence is illustrated in Scheme IV.

Scheme IV

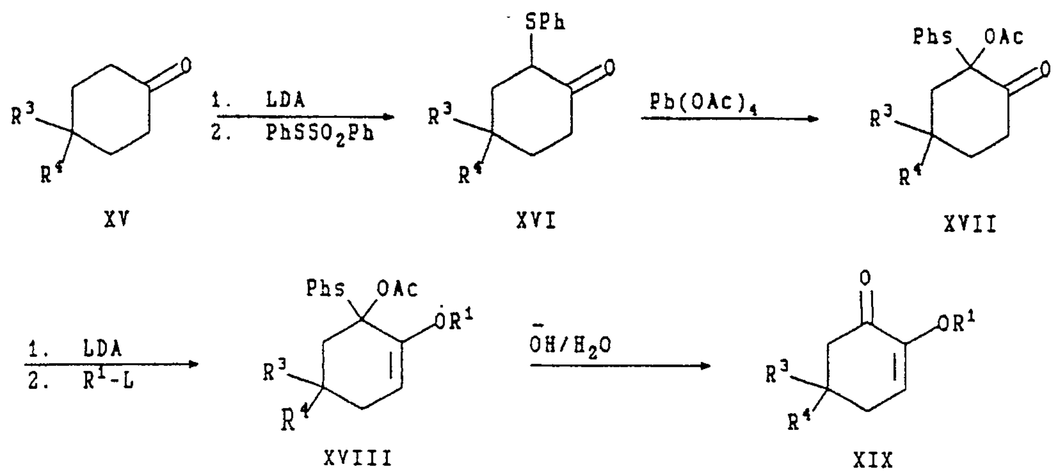


In Scheme IV, $R^3$ and $R^4$ are not both hydrogen but are otherwise as defined under formula I. Cyclohexanone XV, prepared according to procedures described above, is treated first with a base, such as lithium diisopropylamide (LDA), and then with benzenesulfonothioic acid, S-phenyl ester to generate the corresponding α-phenylsulfide compound XVI. A compound of formula XVI is oxidized with lead tetraacetate to provide α-acetoxy-α-phenylthiocyclohexanone derivative of formula XVII, which may be converted to a corresponding silyl enol ether of formula XVIII by first treating with a strong base, such as LDA, followed with a silylating agent $R^1$-L, wherein $R^1$ is a triorganosilyl group and L is a leaving group. $R^1$-L may be, for example, TBS triflate and iodotrimethylsilane. The silyl enol ether of formula XVIII is converted to the corresponding 2-silyloxy-2-cyclohexenone derivative of formula XIX upon base-catalyzed hydrolysis.

Cyclohexenones of formulas XI and XII in which $R^2$ is hydrogen may be converted into their corresponding α-hydroxy derivatives ($R^2 = OH$) by treating the cyclohexenone with a base such as lithium bis(trimethylsilbl)amide, followed by an hydroxylating agent such as the reagent known as MoOPh (Aldrich). The α-hydroxy group may then be protected using conventional protecting groups to provide cyclohexenones of formulas XI and XII in which $R^2$ represents protected hydroxy.

Compounds of formula I may also be prepared from a 7-protected hydroxy-3-heptene-1,5-diyne and an arylsulfocyclohexenone as depicted in Scheme V,

Scheme V

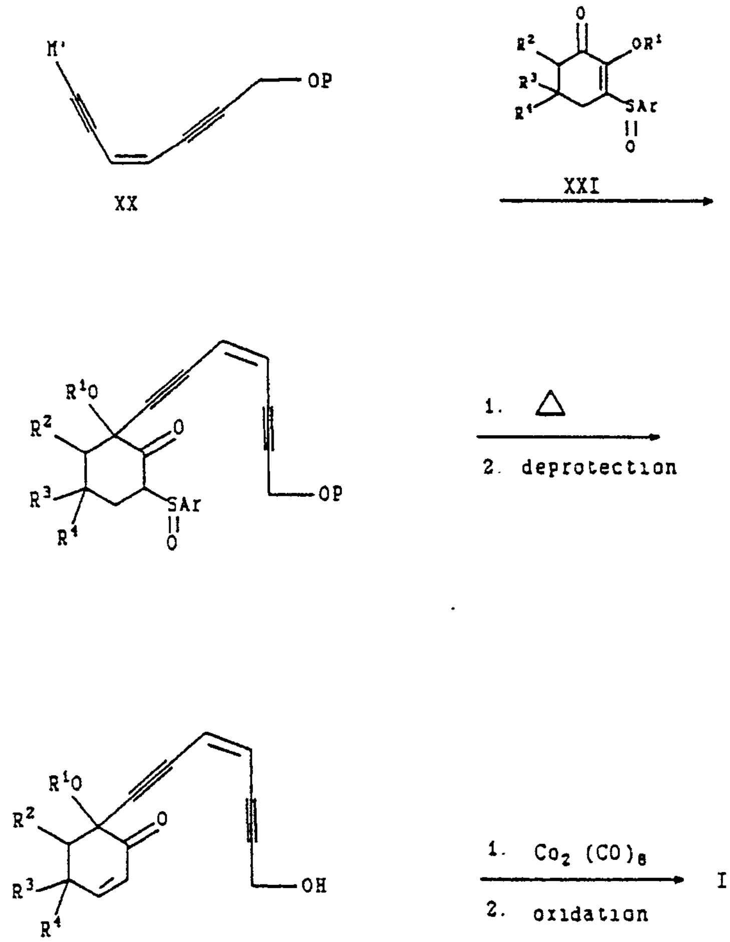


In Scheme V, M', $R^1$, $R^2$, $R^3$, $R^4$ and Ar have the same meaning as previously defined; P is a hydroxy protecting group. The hydroxy protecting group is not particularly limited, the preferred one being tetrahydropyranyl group. Protected diynene alcohol XX may be prepared from cis-1,2-dichloroethylene and protected propargyl alcohol in a manner similar to that described supra for the preparation of the diynene acetal X. The arylsulfocyclohexenone XXI is obtained from the corresponding arylthiocyclohexenone XII upon oxidation with mCPBA.

The condensation of the protected diynene alcohol XX and the arylsulfocyclohexenone XXI is carried out in an inert organic solvent such as tetrahydrofuran at an initial temperature of -78° C. The reaction temperature is allowed to gradually rise to ambient temperature and the reaction is generally completed in a few hours. The product XXII thus obtained is heated in pyridine at about 105° C to cause the elimination of the aryl sulfoxide thus generating the corresponding enone. Deprotection of the hydroxyl group may be accomplished using conventional techniques known in the art; for example, the tetrahydropyranyl group may be removed by acid hydrolysis. The free alcohol of formula XXIII is then converted to the corresponding cobalt complexed diynene aldehyde of formula I by treatment with dicobalt octacarbonyl and oxidation with t-butoxy magnesium bromide and (azodicarbonyl)dipiperdine.

The present invention provides an efficient method for constructing an 8-hydroxy-bicyclo[7.3.1]tridec-4-ene-2,6-diyne-13-one ring system. The products that may be obtained by this process, e.g., compounds of formulas IV and V, may be further elaborated to provide the esperamicin/calichemicin aglycone. Thus, compounds of formulas IV and V are useful intermediates in the synthesis of antitumor compounds belonging to the esperamicin/calichemicin structure class. In addition, compounds of formulas IV and V may be coupled to known antitumor agents, e.g., chlorambucil may be linked to the 8-hydroxy group using an acylating equivalent thereof, such as the acid chloride, to yield active hybrid molecules having more desirable biological activity profile than the parent compound.

Compounds of formula VI in which the protecting groups have been removed and of formula VII wherein

$R^a$ is hydrogen are themselves cytotoxic compounds and are, therefore, useful in inhibiting unwanted rapid proliferation of cells, such as that in the neoplastic process. As therapeutic agents for treating mammalian tumors, these compounds may be administered in the same manners as those suitable for esperamicin and calichemicin. Thus, they may be administered by systemic or topical routes; parenteral administration is the preferred route. The dosage may be similar to that used for esperamicin; but in general, because compounds of the present invention are less cytotoxic than esperamicin, dosage ten to one thousand times that for esperamicin may be tolerated and may be more suitable. The route of administration and the optimal dosage may be readily ascertained by those skilled in the art and will, of course, vary depending on factors such as the type and site of tumor to be treated, and individual patient charateristics, such as extent of the disease, age, weight, and the like.

The invention includes within its scope pharmaceutical compositions containing an effective tumor-inhibiting amount of a compound of formula VI in which the protecting groups have been removed or a compound of formula VII wherein $R^a$ is hydrogen in combination with an inert pharmaceutically acceptable carrier of diluent. Such compositions may be made up in any pharmaceutical form appropriate for the desired route of administration. Examples of such compositions include solid compositions for oral administration such as tablets, capsules, pills, powders and granules; liquid compositions for oral administration such as solutions, suspensions, syrups or elixirs; and preparations for parenteral administration such as sterile solutions, suspensions or emulsions. The pharmaceutical compositions may also be manufactured in the form of sterile solid compositions which can be dissolved in sterile water, physiological saline or some other sterile injectable medium immediately before use.

Furthermore, compounds of formula VI in which the protecting groups have been removed and of formula VII wherein $R^a$ is hydrogen are effective in causing damages to DNA and in double stranded DNA cleavage. They are, therefore, valuable as laboratory reagents for such purposes.

## Biological Activity

Compound of Example 4 was evaluated in vitro against three human colon tumor cell lines: HCT-116, HCT/VM46, and HCT/VP35; the latter two are resistant to teniposide and etoposide, respectively. The in vitro cytotoxicity assay involved growing tumor cells on microtitre plates employing established tissue culture methods. The concentration of the test compound required to inhibit cell growth by 50% ($IC_{50}$) was then determined by four-fold serial dilution technique. In the experiment, etoposide and teniposide were included as positive controls. The results obtained are shown in Table I:

**Table I. Results of In Vitro Cytotoxicity Assay**

| | $IC_{50}$ (μg/ml) | | |
|---|---|---|---|
| Compound | HCT-116 | HCT/VM46 | HCT/VP35 |
| Example 4 | 0.037 | <0.031 | 0.042 |
| | 0.043 | <0.031 | 0.046 |
| | <0.031 | 0.047 | 0.072 |
| Etoposide | 0.101 | 4.24 | 5.14 |
| | 0.128 | 3.57 | 6.29 |
| | 0.140 | 2.08 | 6.75 |
| Teniposide | 0.077 | 0.313 | 0.084 |
| | 0.088 | 0.237 | 0.091 |
| | 0.083 | 0.236 | 0.111 |

The compound of Example 4 was also evaluated against transplantable murine P388 leukemia. $CDF_1$ mice were implanted intraperitoneally with a tumor inoculum of $10^6$ cells of P388 leukemia and treated with various doses of the test compound. Six mice were used for each dose level and 10 mice were treated with saline to serve as control. The text compound was administered intraperitoneally on 5 consecutive days starting on day 1 after tumor implantation. Antitumor activity is expressed as % T/C which is the ratio of mean survival time (MST) for the drug-treated group to the MST of saline-treated control group.

A compound showing a % T/C of 125 or greater is considered to have significant antitumor activity. The

results of P388 test on day 39 of the experiment for compound of Example 4 are provided in Table II. The data indicates this compound as having high activity against P388 leukemia.

Table II. In Vivo Activity Against P388 Leukemia

| Dose (mg/kg/dose) | Med. Survival Time (d) | % T/C | Survival d.5 (39)* |
|---|---|---|---|
| 32 | 7 | 64 | 6/6 |
| 16 | 9.5 | 86 | 6/6 |
| 8 | >39 | >355 | 6/6 (4) |
| 4 | 20.0 | 182 | 6/6 |
| 2 | 16.5 | 150 | 6/6 |
| 1 | 13 | 118 | 6/6 |
| Control | 11 | 100 | 10/10 |

* The number in parenthesis represents the number of surviving mice on day 39.

The following examples are illustrative of the invention only and are not to be construed as limiting the scope of the invention in any manner.

## SPECIFIC EMBODIMENTS

### Preparation of Starting Materials

The structures of the compounds described in this section are provided on separate pages following the Examples section.

### Preparation I. (Z)-7,7-diethoxy-1-trimethylsilyl-3-hepten-1,5-diyne [compound A]

(a) (Z)-5-chloro-1,1-diethoxy-4-pentene-2-yne [compound B]

Neat cis-1,2-dichloroethylene (4.5 ml, 60 mmol) followed by butylamine (8.0 ml, 81 mmol) was added to a solution of copper iodide (0.90 g, 4.73 mmol) and palladium tetrakis(triphenylphosphine) (1 g, 0.86 mmol) in 40 mL of dry benzene stirring at 25° C under argon. Immediately thereafter, a solution of 3,3-diethoxypropyne (5 g, 39 mmol) in 10 mL of benzene was added via cannula. The reaction vessel was wrapped in foil to shield it from light, and the reaction mixture was stirred for 4.2 h at 25° C. The dark brown reaction mixture was filtered by suction through a coarse frit and diluted to approximately 180 ml with diethylether. The solution was washed with 75 mL of water and 120 mL saturated brine, and the organic layer was dried over anhydrous $Na_2SO_4$ and then concentrated in vacuo. The residue was flash chromatographed on $SiO_2$ using 5%, then 10%, and then 15% diethylether/pentane as eluent to provide the desired product as a clear liquid (3.9 g, 55%).

$^1H$ NMR ($CDCl_3$) δ: 6.46 (d, J = 7.5 Hz, 1H), 5.92 (dd, J = 1.5, 7.6 Hz, 1H), 5.45 (d, J = 1.4 Hz, 1H), 3.80 (m, 2H), 3.64 (m, 2H), 1.26 (t, J = 7.0 Hz, 3H).

(b) (Z)-7,7-diethoxy-1-trimethylsilyl-3-heptene-1,5-diyne

A solution of 5-chloro-1,1-diethoxy-4-pentene-2-yne (compound B, 3.8 g, 20 mmol) in 10 mL of benzene was added via cannula to a solution of palladium tetrakis(triphenylphosphine) (1.1 g, 0.95 mmol) and copper iodide (0.47 g, 2.46 mmol) in 20 mL benzene stirring at 25° C under argon. Immediately thereafter, butylamine (4 mL, 40 mmol), followed by trimethylsilylacetylene (5 mL, 40 mmol) was added via syringe.

The reaction vessel was wrapped in foil, and the reaction mixture was stirred at 25° C for 4.25 h. The reaction mixture was poured into 100 mL water and 100 mL diethyl ether and extracted. The aqueous layer was reextracted with 2 x 40 mL of diethyl ether. The combined organic extracts were washed with 50 mL saturated aqueous NaCl, dried over $Na_2SO_4$, and concentrated in vacuo. Flash chromatography over $SiO_2$ using 2%, then 4%, and then 5% diethylether/pentane as eluent provided the title compound (2.7 g, 54%) as a light brown oil.

$^1$H NMR ($CDCl_3$) δ: 5.89 (s, 2H), 5.46 (s, 1H), 3.83-3.75 (m, 2H), 3.69-3.61 (m, 2H), 1.25 (t, J = 7.1 Hz, 6H), 1.40E-4 (s, 9H).

$^{13}$C NMR ($CDCl_3$) δ: 130.6, 111.6, 92.8, 92.0, 79.4, 61.3, 15.2.

Preparation II. (Z)-6-[[(1,1-dimethylethyl)dimethylsilyl] oxy]-6-(7,7-diethoxy-3-heptene-1,5-diynyl)-2-cyclohexenone [compound C]

(a) (Z)-6-[[(1,1-dimethylethyl)dimethylsilyl]oxy]-6-(7,7-diethoxy-3-heptene-1,5-diynyl)cyclohexanone [compound D]

Solid lithium hydroxide monohydrate (3 g, 71.5 mmol) was added to a solution of (Z)-7,7-diethoxy-1-trimethylsilyl-3-heptene-1,5-diyne (compound A, 3.20 g, 12.6 mmol) in 30 mL of tetrahydrofuran and 5 mL water. The reaction mixture was stirred for 4 h and poured into 100 mL of pentane and 50 mL of $H_2O$. The organic layer was washed with 50 mL saturated aqueous NaCl, dried over $Na_2SO_4$, and then concentrated in vacuo by rotary evaporation. Methylene chloride (50 mL) was added, and the solution was again concentrated by rotary evaporation and then placed under high vaccuum for 25 min to provide approximately 3.3 g of a light brown oil which was immediately dissolved in 160 mL of dry tetrahydrofuran. The solution was cooled to -78° C, and then lithium hexamethyl-disilazane (1.0 M, in tetrahydrofuran, 15.5 mL, 15.5 mmol) was added via syringe in one portion. The reaction mixture was stirred for 20 min, and then a solution of 2-tertbutyldimethylsilyloxy-2-cyclohexenone (3.65 g, 6.12 mmol) in 10 mL of dry tetrahydrofuran, which had been precooled to approximately -50° C, was added in one portion via syringe. The reaction mixture was stirred for 1 min, and then all of the cooling baths were removed. The reaction mixture was allowed to stir for 2.5 h and attain ambient temperature (25° C) to generate in situ the lithium enolate. The enolate was quenched with water to provide the corresponding ketone as follows. The reaction mixture was poured into 400 mL of 9:1 ethyl acetate/diethyl-ether and 100 mL of water. The mixture was extracted, and then the aqueous layer was reextracted with 100 ml of 1:1 ethyl acetate/diethyl ether. The combined organic extracts were washed with 50 mL saturated aqueous NaCl, dried over anhydrous $Na_2SO_4$, and concentrated in vacuo. Flash chromatography using 3%, then 4%, and then 5% ethyl acetate/hexane provided 3.50g (72%) of the desired title cyclohexanone as a very faint green oil.

$^1$H NMR ($CDCl_3$) δ: 5.92 (s, 2H), 5.41 (s, 1H), 3.80-3.70 (m, 2H), 3.65-3.55 (m, 2H), 2.88 (dt, J = 13.3, 5.7 Hz, 1H), 2.46 (td, J = 7.7, 12.2 Hz, 1H), 2.27-2.23 (m, 1H), 2.00-1.58 (m, 5H), 1.24 (t, J = 7.08 Hz, 6H), 0.91 (s, 9H), 0.046 (s, 3H), 0.018 (s, 3H).

(b) (Z)-1,6-bis[[[(1,1-dimethylethyl)dimethyl]silyl]oxy]-6-(7,7-diethoxy-3-heptene-1,5-diynyl]cyclohexene [compound E]

Neat tert-butyldimethylysilyl trifluoromethanesulfonate (0.62 mL) was added via syringe to a solution of triethylamine (0.69 mL, 2.72 mmol) and compound D prepared above (0.54 g, 1.33 mmol) in 20 ml of methylene chloride stirring at 25° C under a nitrogen atmosphere. The reaction mixture was stirred for 22.5 h and then poured into 100 mL of methylene chloride and 50 mL of water. The organic layer was dried over anhydrous $Na_2SO_4$ and concentrated in vacuo, and the residue was flash chromatographed on $SiO_2$ using 3% ethyl acetate/hexane to provide the title bis-silyloxy cyclohexene (683 mg, 98%) as a clear liquid.

IR (NaCl, Film): 3046, 2954, 2932, 2890, 2858, 2212, 1660, 1468, 1252 $cm^{-1}$.

$^1$H NMR ($CDCl_3$) δ: 5.88 (d, J = 11.0 Hz, 1H), 5.81 (dd, J = 11.0, 1.3 Hz, 1H), 4.82 (t, J = 3.9 Hz, 1H), 3.79-3.74 (m, 2H), 3.64-3.58 (m, 2H), 2.05-1.99 (m, 4H), 1.81-1.56 (m, 2H), 1.24 (t, J = 7.1 Hz, 6H), 0.94 (s, 9H), 0.87 (s, 9H), 0.21 (s, 3H), 0.18 (s, 3H), 0.17 (s, 3H), 0.16 (s, 3H).

$^{13}$C NMR ($CDCl_3$) δ: 151.1, 121.4, 118.1, 105.0, 102.1, 92.2, 91.5, 82.9, 81.5, 70.2, 61.2, 41.1, 26.1, 24.4, 18.8, 18.5, 18.4, 15.3, -2.8, -3.0, -4.3, -4.4.

(c) (Z)-6-[[(1,1-dimethylethyl)dimethylsilyl]oxy]-6-(7,7-diethoxy-3-heptene-1,5-diynyl]-2-cyclohexenone [compound C]

Selenium dioxide (600 mg, 5.41 mmol) was added to a solution of compound E (1.0 g, 1.98 mmol) in 60 mL of dioxane. The reaction mixture was refluxed for 1.5 h, an additional 300 mg (2.71 mmol) of selenium dioxide was added, and reflux continued for an additional 3 h. The reaction mixture was then poured into 150 mL of ethyl acetate and 100 mL of saturated aqueous $NaHCO_3$. The aqueous layer was reextracted with 50 mL of ethyl acetate. The combined organic layers were dried over anhydrous $Na_2SO_4$, concentrated in vacuo, and purified by flash chromatography on $SiO_2$ using 3% and then 5% ethyl acetate/hexane to provide the title cyclohexenone (620 mg, 77%) as a clear oil.

Anal. calcd. for $C_{23}H_{34}O_4Si$: C, 68.62; H, 8.51.
Found: C, 68.26; H, 8.42.

$^1H$ NMR ($CDCl_3$) δ: 6.93-6.88 (m, 1H), 5.98 (doublet of multiplets, J = 9.49 Hz, 1H), 5.89 (s, 2H), 5.41 (s, 1H), 3.80-3.70 (m, 2H), 3.66-3.55 (m, 2H), 2.82-2.66 (m, 1H), 2.53-2.40 (m, 1H), 2.35-2.16 (m, 2H), 1.24 (t, J = 7.04 Hz, 6H), 0.89 (s, 9H), 0.22 (s, 3H), 0.20 (s, 3H).

$^{13}C$ NMR ($CDCl_3$) δ: 194.4, 150.7, 127.4, 120.5, 120.0, 95.4, 92.4, 92.2, 84.6, 82.5, 73.6, 61.3, 39.0, 26.0, 25.4, 18.5, 15.3, -3.0, -3.1.

Preparation III. Cobalt, hexacarbonyl [μ-[6-](5,6-η:5,6-η)-7,7-diethoxy-3-heptene-1,5-diynyl]-6-[[(1,1-dimethylethyl)-dimethylsilyl]oxy]-2-cyclohexenone]], di(Co-Co), (Z) [compound F]

Solid dicobalt octacarbonyl (0.542 g, 1.58 mmol) was added to a solution of compound C (0.64 g, 1.584 mmol) stirring at 25° C under a nitrogen atmosphere in 28 mL of anhydrous heptane. The reaction mixture was stirred for 2 h and concentrated in vacuo. Flash chromatography using 2%, then 3%, and then 5% ethyl acetate/hexane on $SiO_2$ provided 728 mg (66%) of the desired title cobalt complex as a dark purple oil.

Anal. calcd. for $C_{29}H_{34}O_{10}SiCo_2$:
C, 50.59; H, 4.98; N, 0.0.
Found: C, 50.56; H, 4.99; N, 0.0.

IR (NaCl, Film): 2978, 2956, 2932, 2896, 2858, 2094, 2056, 2028, 1700, 1624, 840 $cm^{-1}$.

$^1H$ NMR (CDCl3) δ: 6.89 and 6.86 (t, 3.91 Hz, 0.5H), 6.78 (d, J = 11.1 Hz, 1H), 5.99 and 5.96 (t, J = 2.0 Hz, 0.5H), 5.87 (d, J = 11.1 Hz, 1H), 5.58 (s, 1H), 3.83-3.75 (m, 2H), 3.68-3.59 (m, 2H), 2.50-2.43 (m, 2H), 2.26 (t, 5.6 Hz, 2H), 1.21 (t, J = 6.9 Hz, 6H), 0.84 (s, 9H), 0.17 (s, 3H), 0.07 (s, 3H).

$^{13}C$ NMR ($CDCl_3$) δ: 199.1 (b), 192.6, 149.9, 137.1, 126.9, 110.5, 101.6, 98.9, 96.0, 85.3, 82.0, 73.6, 63.2, 38.2, 25.7, 24.2, 18.3, 15.0, -3.3, -3.5.

Preparation IV. Cobalt, hexacarbonyl [μ-[6-[(5,6-η:5,6-η)-7-oxo-3-heptene-1,5-diynyl]-6-[[1,1-dimethylethyl)-dimethylsiyl]oxy]-2-cyclohexenone]] di-(Co-Co), (Z) [compound G]

Titanium tetrachloride (0.195 mL, 1.79 mmol) was added via syringe to a solution of the cobalt complexed cyclohexenone compound F (0.41 g, 0.60 mmol) and 1,4-diazabicyclo[2.2.2]octane (67 mg, 0.60 mmol) in 40 mL of methylene chloride stirring at -65° C under a nitrogen atmosphere. The reaction mixture was stirred for 5 min and then poured into 60 mL of methylene chloride and 25 mL of water. The mixture was extracted, and then the aqueous layer was reextracted with 10 mL methylene chloride. The combined organic layers were dried over $Na_2SO_4$, filtered, concentrated by rotary evaporation, and chromatographed to provide the title cobalt complexed aldehyde (301 mg, 82%) as a thick viscous reddish purple semi solid.

Anal. calcd. for $C_{25}H_{24}O_9SiCo_2$:
C, 48.87; H, 3.94; N, 0.00.
Found: C, 48.42; H, 3.82; N, 0.04.

$^1$H NMR ($CDCl_3$) δ: 10.39 (s, 1H), 6.93 (dt, J = 10.1, 4.0 Hz, 1H), 6.82 (d, J = 10.6 Hz, 1H), 6.03 (dt, 10.1, 1.9 Hz, 1H), 5.93 (d, J = 10.8 Hz, 1H), 2.56-2.18 (m, 4H), 0.87 (s, 9H), 0.19 (s, 3H), 0.11 (s, 3H).

$^{13}$C NMR ($CDCl_3$) δ: 198.5-198.1 (b, -CO's), 193.3, 190.8, 150.9, 136.6, 127.4, 111.5, 100.4, 85.2, 73.9, 38.1, 25.9, 24.4, 18.5, -3.15, -3.35.

Preparation V. Alternative method for the preparation of compound E

Following the procedure described in Preparation II (a), a solution of the lithium enolate was prepared from 5 mmol of 7,7-diethoxy-1-trimethylsilyl-3-heptene-1,5-diyne. To this solution stirring at -78° C in 85 mL of solvent was added 1.15 mL (5.0 mmol) of tertbutyldimethyl silyl trifluoromethanesulfonate. The reaction mixture was stirred for 15 min at -78° C and then removed from the cooling bath and allowed to stir for an additional 25 min before being poured into a mixture of 100 mL water, 70 mL ethyl acetate, 25 mL diethyl ether, and 25 mL pentane. The mixture was extracted, and the aqueous layer was reextracted with 100 mL of 50:50 pentane/ethyl acetate. The combined organic extracts were washed with 75 mL saturated aqueous NaCl, dried over $Na_2SO_4$, concentrated in vacuo, and purified by flash chromatography on $SiO_2$ using 2% and then 3% ethyl acetate/hexane as eluent to provide 1.47 g (51%) of the desired silylenol ether identical with material prepared in Preparation II (b).

Preparation VI. Alternative method for the preparation of compound C

Following the procedure of Preparation II (a), a solution of the lithium enolate in 85 mL of solvent was prepared from 5 mmol of 7,7-diethoxy-1-trimethylsilyl-3-heptene-1,5-diyne. To this solution at -78° C was added via syringe over a 2 min period a solution of phenylselenium chloride (0.99 g, 5 mmol) in 6 mL of dry tetrahydrofuran which had been precooled to -78° C. The reaction mixture was stirred for 20 min at -78° C, then the cooling bath was removed, and stirring continued for an additional 20 min. The reaction mixture was poured into 100 mL diethylether/100 mL ethyl acetate/100 mL water and extracted. The aqueous layer was reextracted with 50 mL 1:1 ethylether/ethyl acetate, and the combined organic extracts were washed with 75 mL saturated aqueous NaCl, dried over $Na_2SO_4$, and concentrated in vacuo. Flash chromatography on $SiO_2$ using 4% and then 5% ethyl acetate/hexane provided 1.68 g (57%) of a light brown liquid which was a mixture of phenyselenide, 2-[[(1,1-dimethylethyl)dimethyl]silyl]oxy-2-(7,7-diethoxy-3-heptene-1,5-diynyl)-6-phenylseleno-cyclohexanone [compound H].

The crude mixture of selenides as dissolved in 10 mL of methylene chloride and 0.485 mL (6.0 mmol) of pyridine was added. The solution was cooled in an ice-water bath, and a solution of 0.82 mL (8 mmol) 30% hydrogen peroxide in 1 mL of water was added via syringe in one portion. The reaction mixture was stirred for 5 min, and the ice bath was removed. The reaction mixture was stirred for 25 min, and 0.25 mL of the same hydrogen peroxide (1 mmol) solution was added. The reaction mixture was stirred for 15 min, and 1.57 mL (7 mmol) of $H_2O_2$ solution was added. The reaction mixture was poured into 160 mL methylene chloride, 50 mL saturated aqueous $NaHCO_3$, and 50 mL water. The shaken mixture was filtered by suction to separate the emulsion, and the layers separated. The organic extracts were washed with 50 mL saturated aqueous NaCl, dried over $Na_2SO_4$, and filtered in vacuo. Flash chromatography using 4% EtOAc/Hexane provided the desired enone whose physical properties were consistent with the material obtained in Preparation II (c).

Preparation VII. Alternative method for the preparation of compound C

A 1.0M solution of lithium bis(trimethyl)silylamide in THF (28.7 mL, 28.77 mmol) was added to 30 mL of dry THF stirring under $N_2$, and the solution was cooled to -10° C. A solution of 1.54 g (13.7 mmol) 1,2-cyclohexanedione in 7 mL of THF was added in a slow stream via syringe. The reaction mixture turned dark-reddish brown. The reaction mixture was stirred for 15 min at -10° C and then cooled to -50° C. A solution of 4-methylphenyl 4-methyl-benzenethiosulfonate (3.5 g, 14.2 mmol) in 10 mL of THF at -50° C was added in one portion via syringe. The reaction mixture was stirred for 2 h at -50° C and allowed to warm at ambient temperature until approximately 0° C, and then 100 mL of 0.1N HCl was added. The mixture was extracted with 300 mL and then 100 mL of diethyl ether, and the combined organic extracts were dried over anhydrous sodium sulfate. Concentration in vacuo provided a yellow solid which was purified by flash chromatography over silica gel using $CH_2Cl_2$, 10% EtOAc/Hexane, and then 3% $MeOH/CH_2Cl_2$ as eluent. The desired product, 2-hydroxy-3-[(4-methylphenyl)thio]-2-cyclohexenone [compound Iy], was isolated as a white solid (1.939 g, 60%).

Anal. calcd. for $C_{13}H_{14}O_2S$:

C, 66.64; H, 6.02; N, 0.00.

Found: C, 66.63; H, 6.10; N, 0.00.

IR (KBr): 3372, 3054, 2954, 2920, 2870, 2834, 1642, 1600, 820, 656, 628 $cm^{-1}$.

$^1H$ NMR ($CDCl_3$) $\delta$: 7.38 (d, J = 8.1 Hz, 2H), 7.16 (d, J = 8.2 Hz, 2H), 6.47 (s, 1H, -OH), 2.44 (t, J = 6.2 Hz, 2H), 2.35 (s, 3H), 2.17 (t, J = 5.9 Hz, 2H), 1.87 (m, 2H).

$^{13}C$ NMR ($CDCl_3$) $\delta$: 191.1, 143.0, 140.3, 136.0, 133.5, 130.6, 126.3, 35.4, 28.3, 22.8, 21.5.

Tert butyldimethylsilyltrifluoromethanesulfonate (TBSOTf) (20.75 mL, 90.03 mmol) was added via syringe to a solution of compound Iy (17.64 g, 75.28 mmol) and $Et_3N$ (15.7 mL, 112.92 mmol) stirring in 250 mL of methylene chloride under a nitrogen atmosphere at 2° C. After 5 min, the cooling bath was removed, and the reaction mixture was allowed to stir for 22 h. An additional 0.78 mL (5.59 mmol) of $Et_3N$ followed by 0.865 mL (3.76 mmol) TBSOTf was added, and the reaction mixture was stirred for 2 h more. The reaction mixture was poured into 200 mL of water and 50 mL $CH_2Cl_2$ and extracted. The organic layer was washed with saturated aqueous brine, dried over $Na_2SO_4$, and concentrated in vacuo. Purification by flash chromatography over $SiO_2$ using a 0-10% EtOAc/hexane gradient as eluent provided 21.22 g (81%) as an off-white solid of 2-TBSoxy-3-[(4-methylphenyl)thio]-2-cyclohexenone [compound J].

$^1H$ NMR ($CDCl_3$) $\delta$: 7.38 (d, J = 8.06 Hz), 2H), 7.15 (d, J = 7.9 Hz, 2H), 2.35 (s, 3H), 2.38-2.34 (m, 2H), 2.12 (t, J = 6.05 Hz, 2H), 1.82 (m, 2H), 1.00 (s, 9H), 0.21 (s, 6H).

A solution of 8.64 g (40 mmol) 80-85% pure MCPBA in 125 mL of dichloromethane at 25° C was added via pipette to a solution of 12.7 g (36.4 mmol) compound J in 600 mL of dichloromethane at -78° C. The reaction mixture stirred for 1.75 h at -78° C and poured into 1,100 mL of diethyl ether and 500 mL sat aq. $Na_2SO_3$. After extraction, the organic layer was washed successively with 300 mL and 200 mL portions of saturated aqueous $NaHCO_3$ and then dried over sodium sulfate. After removal of the solvent in vacuo, the crude product was purified by flash chromatography on $SiO_2$ using 10% and then 20% EtOAc/Hexane as eluent to provide 12.2017 g (92%) of the corresponding sulfoxide [compound K] as viscous, light-yellow oil.

Anal. calcd. for $C_{19}H_{28}O_3SSi$:

C, 62.59; H, 7.49; N, 0.00.

Found: C, 62.24; H, 7.49; N, 0.06.

IR (NaCl, Film): 2954, 2930, 2886, 2858, 1694, 1610, 1080 $cm^{-1}$.

$^1H$ NMR ($CDCl_3$) $\delta$: 7.48 (d, J = 8.3 Hz, 2H), 7.28 (d, J = 8.2 Hz, 2H), 2.82-2.74 (m, 1H), 2.55-2.46 (m, 1H), 2.38 (s, 3H), 2.38-2.29 (m, 1H), 2.05-1.86 (m, 3H), 0.98 (s, 9H), 0.32 (s, 3H), 0.20 (s, 3H).

$^{13}C$ NMR ($CDCl_3$) $\delta$: 195.5, 142.6, 141.6, 140.5, 130.6, 124.3, 38.3, 26.1, 22.4, 21.5, 19.2, 18.5, -3.4, -4.0.

Lithium bis(trimethylsilyl)amide (40 ml of 1.0 M solution in THF) was added via syringe to a solution of 7.13 g (40.0 mmol) of 7,7-diethoxy-3-heptene-1,5-diyne stirring in 250 mL dry THF at -78° C under a nitrogen atmosphere. The dark solution was stirred for 30 min, and then a solution of 12.15 g (33.3 mmol) of compound K which had been precooled to -78° C was added via cannula over 5 min. The reaction mixture was removed from the cooling bath and allowed to stir at ambient temperature for 1.6 h. The reaction mixture was poured into 500 mL 1N HCl and 1 L 1:1 ether:ethyl acetate and extracted. The aqueous layer was reextracted with two 150 mL portions of the same solvent, and the combined organic extracts were washed with 200 mL saturated aqueous NaCl and then dried over anhydrous $Na_2SO_4$. Purification by flash chromatography over $SiO_2$ using a gradient of 10-25% EtOAc/Hexane as eluent provided 15.5 g (86%) of a viscous brown liquid which was the desired product as a mixture of diastereomers [compound L].

IR (NaCl, Film): 2930, 2858, 2214, 2256, 1736, 1652, 1798, 1086, 1052 $cm^{-1}$.

A solution of 12.02 g (22.1 mmol) of compound L and 7.4 g (44.3 mmol) of 2-mercaptobenzothiazole in 100 g of heptyne was heated at reflux for 50 min, allowed to cool, and then poured into 400 mL diethyl ether and 400 mL water. The layers were separated and the organic layer dried over anhydrous sodium sulfate. Removal of most of the ether by rotary evaporation caused deposition of a brown precipitate which was removed by suction filtration and discarded. Purification of the filtrate by flash chromatography over silica gel in the usual manner provided 4.47 g (50%) of the desired enone which had the physical characteristics described previously.

Preparation VIII. Alternative method for the preparation of compound G

(a) (Z) 5-chloro-1-(2-tetrahydropyranyloxy)-4-pentene-2-yne (compound M)

Tetrahydrofuran (degassed, 600mL) was added via cannula to a flask containing 7.55g (39.6 mmol) CuI and 5.93g (5.13mmol) tetrakis(triphenylphosphine)palladium(0) stirring under an argon atmosphere. Neat cis 1,2-dichloroethylene (50g, 516mmol) was added via syringe followed by 68mL (688mmol) of butylamine. 2-(3-propynyloxy)tetrahydropyran (48g, 344mmol) was added dropwise over 10 min. After the addition was complete the reaction mixture was stirred for 10 min and then cooled in an external ice water bath for 40 min. The cooling bath was removed and the rection mixture allowed to stir for 4h at ambient temperature (25° C). Air was bubbled through the reaction mixture for 15min and then the reaction mixture was filtered by suction through a glass frit using pentane then ether washes for transfer. The reaction was taken up in ethyl acetate and washed twice with water. The aqueous layers were reextracted with ethyl acetate and the combined organic layers dried over sodium sulfate, filtered and concentrated in vacuo. Flash chromatography over silica gel using 2.5% then 4% ethyl acetate/hexane as eluent provided 48.74g (71%) of clear, slightly reddish liquid:

$^1$H NMR ($CDCl_3$) δ 6.33 (d,J=7.5Hz,1H), 5.84 (m,1H), 4.80 (m,1H), 4.36(m,2H), 3.78 (m,1H), 3.48 (m,1H), 1.80-1.40 (m,6H).

$^{13}$C NMR ($CDCl_3$) δ 128.7, 111.6, 96.7, 93.5, 79.6, 62.0, 54.5, 30.2, 25.3, 19.0.

IR (NaCl film) 3084, 3026, 2944, 2870, 2854, 1592 cm$^{-1}$.

Anal. calcd. for $C_{10}H_{13}ClO_2$: C, 59.86; H, 6.53. Found: C, 59.74; H, 6.44; N, 0.03.

(b) (Z) 7-(2-tetrahydropyranyloxy)-1-(trimethylsilyl)-3-heptene-1,5-diyne (compound N)

Degassed anhydrous tetrahydrofuran (500mL) was added to 5.86g (4.32mmol) solid tetrakis-(triphenylphosphine) palladium(0) and copper (I) iodide (5.17g, 27.1mmol) stirring under argon. A solution of the vinyl chloride (compound M of step (a) 45.38g, 226.1mmol) in 100mL of dry tetrahydrofuran was added via cannula followed immediately by the addition of 45mL (455mmol) neat butylamine. The flask was wrapped in aluminum foil to exclude light and then 41.6 mL (294mmol) of trimethylsilyl acetylene was added via syringe over 4min. After about 10 min the reaction became very warm and was cooled in an ice water bath for 3 min. The cooling bath was then removed and the reaction allowed to stir for 4h at ambient temperature. Air was bubbled through the reaction for 15 min and then the reaction was filtered by suction through a glass frit using pentane then ether washes for transfer. The reaction was diluted with ether and washed with five 500ml portions of water. The aqueous washes were reextracted with a small amount of diethyl ether and the combined organic extracts were dried over anhydrous $Na_2SO4$, filtered, and concentrated on a rotary evaporator. Flash chromatography over $SiO_2$ using 2.5% to 4% ethyl acetate/hexane as eluent provided 52.86g (89%) of liquid as the desired product:

$^1$HNMR ($CDC1_3$) δ 5.83 (m,2H), 4.86 (bs, 1H), 4.44 (ABq, Jab=14Hz,2H), 3.82 (m,1H), 3.52 (m,1H), 1.89-1.49 (m,6H), o.33(s,9H).

$^{13}$C NMR ($CDC1_3$) δ 120.1, 119.7, 103.0, 101.7, 96.5, 93.4, 83.0, 61.8, 54.6, 30.2, 25.3, 18.9, -0.21.

IR (NaCl film) 2956, 2144, 844 cm$^{-1}$.

Anal.calcd for $C_{15}H_{22}O_2Si$: C, 68.65; H, 8.45; N, 0.00. Found: C, 68.79; H, 8.35. N, 0.06.

(c) (Z) 7-(2-tetrahydropyranyloxy)-3-heptene-1,5-diyne (compound-O)

Lithium hydroxide monohydrate (22.55g, 0.54mol) was added in one portion to a solution of 21.62g (82.38 mmol) silyl diynene (compound N of step (b)) in 240 mL tetrahydrofuran and 40mL water stirring at 25° C. The reaction was stirred for 1.58h and then diluted with 1:1 ether:hexane and water. The aqueous layer was reextracted with three portions of ether and then the combined organic extracts were dried over anhydrous sodium sulfate. Flash chromatography over silica gel using a gradient of 2.5 to 10% ethyl acetate/hexane as eluent provided 15.69g (98%) of brown liquid:

$^1$HNMR ($CDCl_3$) δ 5.92 (d,J=11.OHz,1H), 5.78 (dd,J=11.1, 2.2Hz,1H), 4.87 (m,1H), 4.45 (tallmultiplet, 2H), 3.83 (m,1H), 3.52 (m,1H), 3.30 (d,J=2.1Hz,1H), 1.79-1.48 (m,6H).

$^{13}$CNMR ($CDCl_3$) δ 121.2, 118.7, 96.6, 93.5, 84.7, 82.7, 80.5, 62.0, 54.6, 30.2, 25.4, 19.0.

IR (NaCl film) 3288, 2944, 2870, 2854, 2096 cm-1.

Anal calcd for $C_{12}H_{14}O2$= C, 75.76; H, 7.42. Found= C, 75.22; H, 7.30.

(d) (Z) 6-[[(1,1-dimethylethyl)dimethylsilyloxy]-6-[7-(2-tetrahydropyranyloxy)-3-heptene-1,5-diynyl]-2-cyclohexenone (compound P)

A 1.OM solution of lithium bis(trimethylsilylamide) in tetrahydrofuran (89mL, 89mmol) was added via syringe to a solution of 15.4g (80.9mmol) diynene (compound O of step (c)) in 500mL of tetrahydrofuran stirring at -78° C under an atmosphere of nitrogen. The reaction was stirred for 35min and then a solution of 24.6g (67.46mmol) sulfoxide ketone (compound K) in 100mL of tetrahydrofuran at -70° C was added via cannula. The cooling bath was removed and the reaction was allowed to stir at ambient temperature for 2h (gradually reaching 25° C). The reaction was quenched with saturated aqueous ammonium chloride and extracted with two portions of 1:1 ethyl acetate:ethyl ether. The combined organic extracts were washed with saturated aqueous sodium bicarbonate then saturated sodium chloride, dried over sodium sulfate, and concentrated in vacuo. Purification by flash chromatography over silica gel using a gradient of 2.5 to 20% ethyl acetate/hexane as eluent provided 27.3g (73%) of viscous oil which was the desired keto sulfoxides as a mixture of diastereomers.

A solution of 27.3g (49.2mmol) of the sulfoxide from above in 540mL of pyridine was heated at 105° C for 1.75h and then diluted with toluene (several portions were added to help remove the pyridine azeotropically) and concentrated on a rotary evaporator. The crude product was purified by flash chromatography using 5 then 10% ethyl acetate/hexane as eluent to provide 18.0g (88%) of a light yellow oil which was the desired product contaminated with trace amounts of sulfur byproducts. This material was used directly in the next alcohol deprotection reaction:

$^1$HNMR ($CDCl_3$) $\delta$ 6.89-6.82 (m,1H), 5.96 (dd,J=10.9,1.2Hz,1H), 5.87-5.77 (m,2H), 4.78 (m,1H), 4.46-4.31 (m,2H), 3.81(m,1H), 3.51 (m,1H), 2.70-2.64 (m,1H), 2.47-2.39 (m,1H), 2.29-2.14 (m,2H), 1.81-1.49 (m.6H), 0.68 (s,9H), 0.20 (s,3H), 0.18 (s,3H).

(e) (Z) 6-[[(1,1-dimethylethy)dimethylsilyl]-6-[(7-hydroxy)-3-heptene-1,5-diynyl]-2-cyclohexenone (compound Q)

Para-toluenesulfonic acid (1.21g, 6.36mmol) was added to a solution of the tetrahdropyranyl ether (compound P of step (d) 18.0g, 43.3mmol) in 200mL of methanol stirring at 25° C. The reaction was stirred for 30 min and then concentrated on a rotary evaporator. The crude oil was taken up in ethyl acetate and washed with saurated aqueous $NaHCO_3$ and then saturated aqueous NaCl. The organic extracts were dried over anhydrous sodium sulfate and then concentrated in vacuo. Purification by flash chromatography over silica gel using a gradient of 5%-20% ethyl acetate/hexane as eluent provided 10.75g (75%) of the desired product as a light yellow oil:

$^1$HNMR ($CDCl_3$) $\delta$ 6.91 (m,1H), 5.98 (dt,J=10.3,1.9Hz,1H), 5.88 (dt,J=10.8,0.6Hz,1H), 5.80 (d,J=10.7Hz,1H), 4.40 (d,J=6.2Hz,2H), 2.51-2.20 (m,4H), 1.59 (s.1H), 0.85 (s,9H), 0.20 (s,3H), 0.18 (s,3H).

$^{13}$C NMR ($CDCl_3$) 194.3, 150.7, 127.0, 121.0, 119.0, 95.9, 94.3, 84.8, 82.6, 72.9, 51.3, 38.3, 25.5, 24.5, 18.0, -3.5, -3.6.

(f) Cobalt, hexacarbonyl [μ-[6-[(5,6η:5,6η)-(Z) 6-[[(1,1-dimethylethy)dimethylsilyl]-6-[(7-hydroxy)-3-heptene-1,5-diynyl]-2-cyclohexenone, di(Co-Co) (compound R)

Octacarbonyldicobalt (Alfa, 4.41g, 12.9mmol) was added in one portion to a solution of 4.27g (12.9 mmol) enone alcohol (compound Q of step (e)) stirring in 170 mL of dichloromethane at 25° C under an atmosphere of nitrogen. The reaction was stirred for 2h and then concentrated on a rotary evaporator. Purification by flash chromatography over silica gel using a gradient of 5 to 20% ethyl acetate/hexane provided 6.18g (78%) of the desired product as a purple oil:

$^1$H NMR ($CDCl_3$) $\delta$ 6.91 (m,1H), 6.72 (d,J=10.7Hz,1H), 6.01 (broad doublet,J=10.1Hz,1H), 5.77 (d,J=10.6Hz,1H), 4.85 (d,J=6.5Hz,2H), 2.56-2.30 (m,2H), 2.27 (t,J=5.6Hz,2H), 1.50 (bs,1H), 0.85 (s,9H), 0.19 (s,3H), 0.074 (s,3H).

A slower eluting minor isomer (1.29g, 16%) was also isolated and is the cobalt complex of the other acetylene of the diynene.

(g) Compound G

A 2.0 M solution of ethylmagnesium bromide in tetrahydrofuran (6.50mL, 13mmol) was added dropwise to a solution of t-butonal (1.29mL, 13.5mmol) in 30 mL of dry tetrahydrofuran at 0° C. The reaction was stirred for 15min and then the cooling bath was removed. After 5 min a solution of the cobalt complexed alcohol (compound R of step (f) 6.18g, 10.03 mmol) was added via cannula. A solution of 3.39g (13.4mmol)

1,1'-(azodicarbonyl)dipiperidine in 30 mL dry tetrahydrofuran was added dropwise via cannula. After the addition was complete the reaction was poured into a saturated aqueous brine solution and extracted. The organic layer was washed with saturated aqueous sodium bicarbonate and then saturated aqueous brine. The combined aqueous washes were extracted once with 1:1 ethyl acetate: ethyl ether and then the combined organic extracts were dried over anhdrous sodium sulfate and concentrated in vacuo. Flash chromatography using 10% ethyl acetate/hexane provided 5.57g (89%) of reddish purple viscous oil.

Preparation IX. Preparation of dimethylphenylthiol aluminum

A solution of 1.0 mL of 2.0 M trimethylaluminum in hexanes was added dropwise over 0.5 min to a solution of 0.2054 mL (2.0 mmol) thiophenol stirring in 2 mL dry hexane under a nitrogen atmosphere in an ice water cooling bath.

The reaction mixture was stirred for 30 min, and then 6 mL of dry tetrahydrofuran was added via syringe.

Example 1. Cobalt, hexacarbonyl[μ-[(6,7-η)-1-[[(1,1-dimethylethyl) dimethylsilyl]oxy]-8-hydroxy-10-phenylthiobicyclo[7.3.1]tridec-4-ene-2,6-diyn-13-one]] di (Co-Co)

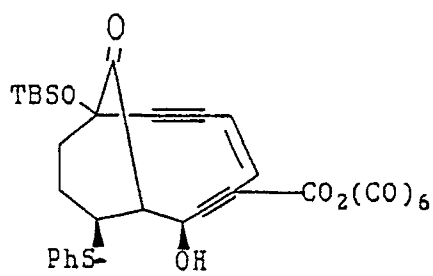

A previously prepared stock solution of dimethyl(phenylthio)aluminum (6.85 ml, 1.49 mmol) was added in one portion via syringe to a solution of enone cobalt complex aldehyde (compound G, 297 mg, 0.483 mmol) in 11 mL of dry tetrahydrofuran stirring under a nitrogen atmosphere at -50° C. The reaction mixture was stirred for 15 min and then cooled to -78° C. The reaction mixture was then allowed to warm to -50° C over 90 min, and neat titanium isopropoxide (1.0 mL, 3.34 mmol) was added thereto in one portion via syringe. The reaction mixture was stirred for 15 min at a temperature between -50° C and -45° C, and then an additional 2.0 mL (6.68 mmol) of neat titanium isopropoxide was added. The reaction mixture was stirred for 15 min between -50° and -45° C and then 20 min between -45° and -40°, and then an additional 2.0 mL (6.68 mmol) of titanium isopropoxide was added. The reaction mixture was stirred for 15 min between -40° C and -30° C and then recooled to -65°. The reaction mixture was allowed to warm to -55° over 30 min, and then the cooling bath was removed. The reaction mixture was stirred for 20 min at ambient temperature and then poured into 300 ml of ethyl acetate and 100 mL of water and extracted. The aqueous layer was reextracted with 100 ml of ethyl acetate, and the combined organic layers were washed with 100 ml saturated aqueous NaCl solution and then dried over anhydrous $Na_2SO_4$. After filtration and concentration in vacuo, the crude product was purified by flash chromatography on $SiO_2$ to provide four fractions of material described in their order of elution from the column:

Fraction 1 contained 56 mg (16%) of purple viscous oil which was an aldehyde resulting from simple conjugate addition of phenylmercaptan; fraction 2 contained 68.2 mg (23%) of pure recovered starting material; and fraction 3 contained a 6:4 mixture of the desired product and starting material (59 mg, 11% desired, 6% starting material).

Fraction 4 provided 142 mg (41%) of the desired title compound as a reddish-purple foam.

IR (KBr): 3442, 3060, 2954, 2930, 2894, 2858, 2096, 2060, 2029, 1730, 1080, 838, 780, 744 $cm^{-1}$.

$^1H$ NMR ($CDCl_3$) δ: 7.50-7.47 (m, 2H), 7.36-7.24 (m, 3H), 7.03 (d, J = 9.9 Hz, 1H), 5.79 (d, J = 9.9 HZ, 1H), 5.29 (bt, J = 7.5 Hz, 1H), 4.32 (bs, 1H), 2.77 (d, J = 9.2 Hz, 1H), 2.51-2.38 (m, 2H), 2.28-2.27 (m, 1H), 1.98-1.93 (m, 1H), 1.38 (d, J = 7.5 Hz, 1H, -OH), 0.83 (s, 9H), 0.19 (s, 3H), 0.13 (s, 3H).

$^{13}C$ NMR ($CDCl_3$) δ: 199.1 (b), 198.3, 142.4, 133.7, 129.7, 128.4, 110.5, 99.2, 97.4, 92.1, 82.6, 69.4, 62.6, 48.5, 37.1, 25.9, 23.4, 18.5, -2.6, -2.9.

Example 2. 8-hydroxy-bicyclo[7.3.1]trideca-4,9-diene-2,6-diyn-13-oneBicyclo[7.3.1]trideca-4,9-diene-2,6-diyn-8-hydroxy-13-one, 1,[[(1,1-dimethylethyl)dimethylsilyl]oxy]

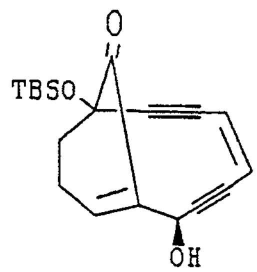

(a) Preparation of 1-[[(1,1-dimethylethyl))dimethylsilyl]oxy]-8-hydroxy-10-phenylthio-bicyclo[7.3.1]tridec-4-ene-2,6-diyn-13-one

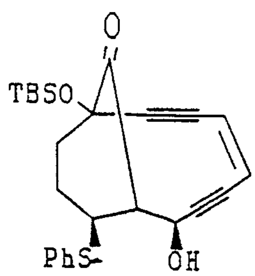

Iodine crystals (12 mg, 0.095 mmol) were added to a solution of cobalt complex reaction product of Example 1 (19 mg, 0.026 mmol) in 5 mL of dry benzene stirring under a nitrogen atmosphere at 25° C. The reaction mixture was stirred for 2 h, concentrated slightly in vacuo, and then flash chromatographed on $SiO_2$ using 4% ethyl acetate/hexane as eluent to provide 6 mg (53%) of the desired decomplexed substrate as a clear oil.

FAB Ms (NOBA): $M^+$ 438.

IR Neat (NaCl, Film): 3484, 3060, 2954, 2958, 2208(w), 1714, 1584 $cm^{-1}$.

$^1H$ NMR ($CDCl_3$) δ: 7.48-7.38 (m, 2H), 7.36-7.31 (m, 3H), 5.92 (s, 2H), 5.25 (dd, J = 11.0, 4.5 Hz, 1H), 4.43 (d, J = 11.0 Hz, 1H, -OH), 4.05 (dt, J = 5.5, 9.8 Hz, 1H), 2.81 (dd, J = 10.1, 4.6 Hz, 1H), 2.43 (m, 1H), 2.29 (m, 1H), 2.10 (m, 1H), 1.91 (m, 1H), 0.88 (s, 9H), 0.19 (s, 3H), 0.17 (s, 3H).

$^{13}C$ NMR ($CDCl_3$) δ: 207.1, 134.7, 132.6, 129.8, 129.0, 125.1, 123.6, 99.6, 98.0, 92.9, 85.0, 74.4, 65.7, 59.1,

43.1, 34.7, 26.9, 25.9, 18.4, -2.9, -3.0. (b) Preparation of 1-[[(1,1-dimethylethyl)dimethylsilyl]-oxy]-8-hydroxy-bicyclo[7.3.1]trideca-4,9-diene-2,6-diyn-13-one

Solid sodium periodate (120 mg, 0.56 mmol) was added to a solution of the product of step (a) above (5 mg, 0.011 mmol) in 5 mL methanol and 2 mL water at 25° C. The reaction mixture was stirred for 10 min, and 1 mL of water was added to dissolve the precipitate, and the stirring continued for 90 min. An additional 149 mg (0.70 mmol) of sodium periodate was added, and the reaction mixture stirred for 45 min and extracted with 50 mL of methylene chloride and 5 mL of water. The aqueous layer was reextracted with 10 mL of methylene chloride. The combined organic layers were dried over anhydrous $Na_2SO_4$, concentrated in vacuo, and flash chromatographed on $SiO_2$ using 10% ethyl acetate/hexane as eluent to provide two fractions:

Fraction 1 provided less than 1 mg of a minor, faster eluting side product. Fraction 2 provided 2 mg (56%) of the desired title compound as a white solid.

FAB MS (NOBA): (M+H) 329.

IR Neat (NaCl) 3356, 2952, 2928, 2856, 1712, 1690, 1414, 782 $cm^{-1}$.

$^1H$ NMR ($CDCl_3$) δ: 6.37 (bs, 1H), 5.84 (s, 2H), 5.22 (d, J = 10.8 Hz, 1H), 4.82 (d, J = 10.8 Hz, 1H, -OH), 2.51-2.47 (m, 2H), 2.28-2.25 (m, 1H), 2.16-2.10 (m, 1H), 0.91 (s, 9H), 0.22 (s, 3H), 0.19 (s, 3H).

$^{13}C$ NMR ($CDCl_3$) δ: 196.8, 139.4, 137.2, 124.8, 123.1, 101.4, 96.3, 93.0, 87.7, 74.6, 69.2, 34.6, 26.0,

24.8, 18.5, -2.8, -3.1.

Example 3. Alternative preparation of compound of Example 2

Solid 3-chloroperbenzoic acid, (mCPBA, 10.3 mg, .059 mmol) was added to a solution of the product of Example 1 (23.4 mg, .032 mmol) in 10 ml of methylene chloride at 25° C. The reaction mixture was stirred for 15 min, and an additional 14.9 mg (.086 mmol) was added. The reaction mixture was stirred for another hour during which 6.2 mg (.035 mmol) more of mCPBA was added. The reaction mixture was poured into approximately 20 ml of methylene chloride and 10 ml of saturated solution of $NaHCO_3$. The aqueous layer was extracted with methylene chloride and the organic layer washed with saturated solution of NaCl, and the combined organic layer was dried over anhydrous $Na_2SO_4$ and concentrated in vacuo. Flash chromatography on $SiO_2$ using 5% ethylacetate/hexane as eluent provided 4.6 mg (43%) of the desired product as an off-white solid.

FAB MS (NOBA): (M+H) 329.

IR Neat (NaCl): 3356, 2952, 2928, 2856, 1712, 1690, 1414, 782 $cm^{-1}$.

$^1H$ NMR ($CDCl_3$) $\delta$: 6.37 (bs, 1H), 5.84 (s, 2H), 5.22 (d, J = 10.8 Hz, 1H), 4.82 (d, J = 10.8 Hz, 1H, -OH), 2.51-2.47 (m, 2H), 2.28-2.25 (m, 1H), 2.16-2.10 (m, 1H), 0.91 (s, 9H), 0.22 (s, 3H), 0.19 (s, 3H).

$^{13}C$ NMR Partial ($CDCl_3$) $\delta$: 139.4, 137.2, 124.8, 123.1, 93.0, 87.7, 69.2, 34.6, 29.9, 26.0, 24.8, -2.8, -3.1.

Example 4. 1,8-Dihydroxy-bicyclo[7.3.1]trideca-4,9-diene-2,6-diyn-13-one

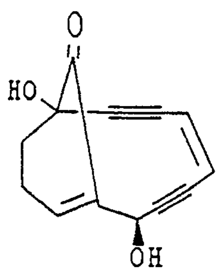

Trifluromethanesulfonic acid (2 drops from a 22 gauge needle, 0.010 mL, 0.11 mmol) was added to a solution of 8-hydroxy-1-TBSoxy-bicyclo[7.3.1]trideca-4,9-diene-2,6-diyn-13-one (product of Example 2, 12.2 mg, 0.040 mmol) in 7 mL of dichloromethane containing 800 mg of 4A molecular sieves stirring at 25° C. The reaction mixture was stirred for 10 minutes, diluted with 50 mL dichloromethane, and washed with 50 mL of saturated aqueous sodium bicarbonate. The organic layer was concentrated in vacuo. The crude product thus obtained was combined with the crude product from a similar experiment in which 2.5 mg of the enone alcohol was utilized. Flash chromatography of the combined crude product over silica gel using 10% and then 20% ethyl acetate/hexane as eluent provided 8.6 mg (83%) of the desired product as a white stable solid.

MS: m/e 214.

$^1H$ NMR ($CDCl_3$) $\delta$: 6.51 (m, 1H), 5.84 (s, 2H), 5.23 (d, J = 11.2 Hz, 1H), 4.38 (d, J = 11.2 Hz, 1H, -OH), 3.93 (s, 1H, -OH), 2.58-2.51 (m, 2H), 2.46-2.39 (m, 1H), 2.13-2.01 (m, 1H).

Compound A

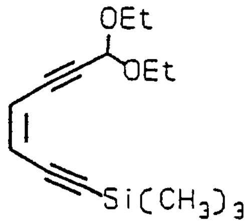

Compound B

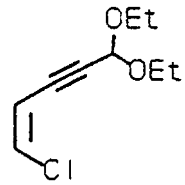

Compound C

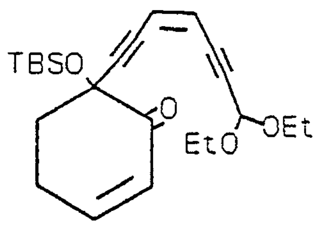

Compound D

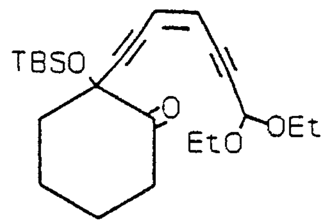

Compound E

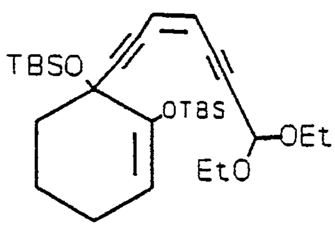

Compound F

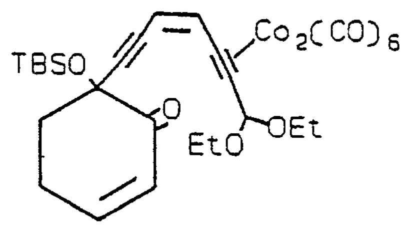

Compound G

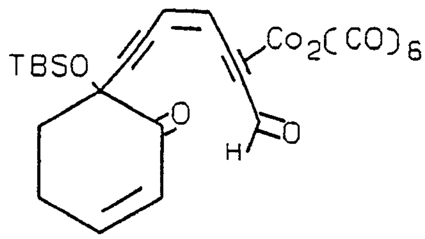

Compound H

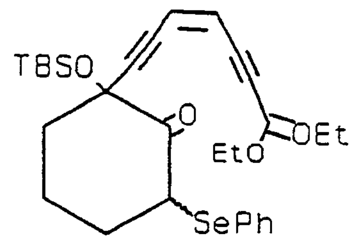

Compound Iy

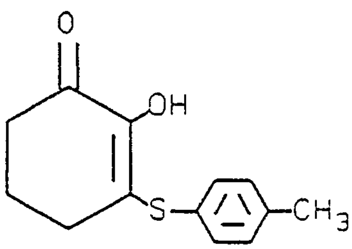

Compound J

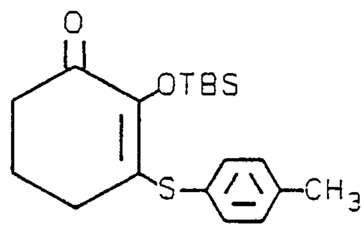

Compound K

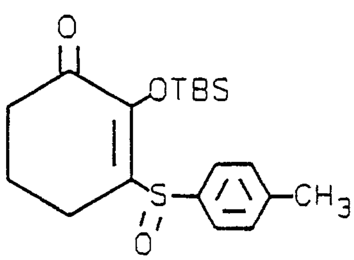

Compound L

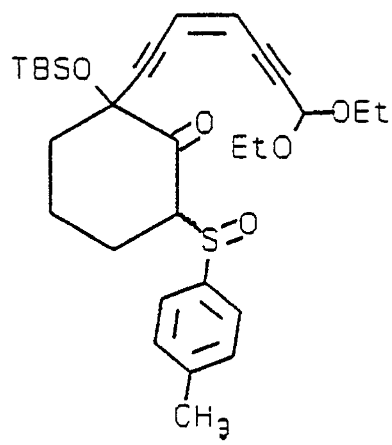

Compound M

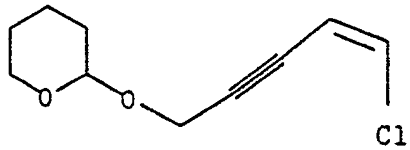

Compound N

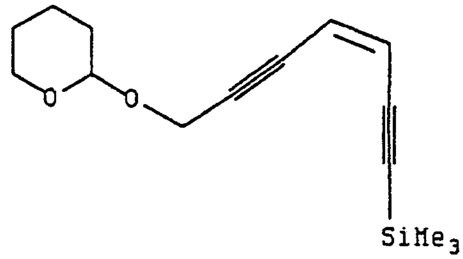

Compound O

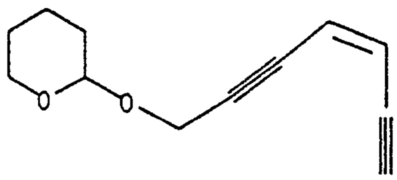

Compound P

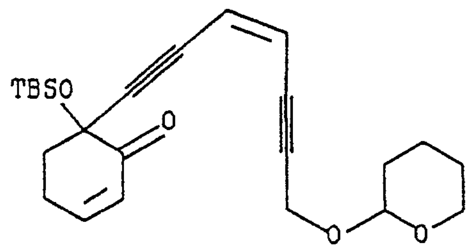

Compound Q

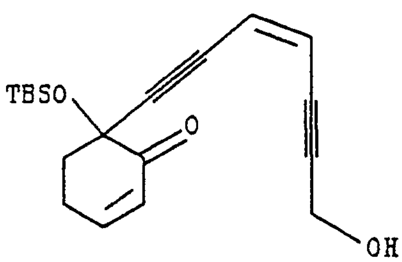

Compound R

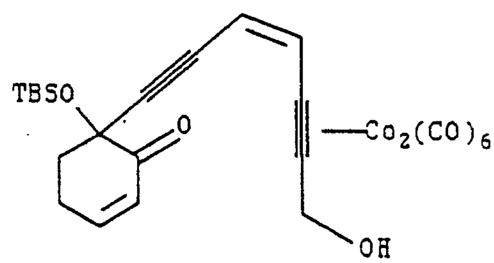

## Claims

**1.** A process for preparing a compound having the formula

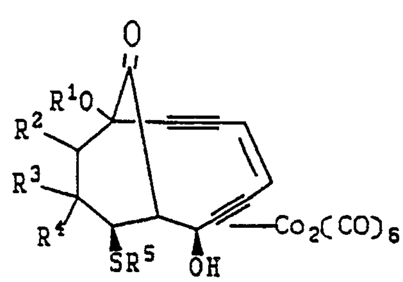

wherein

$R^1$ is hydroxy protecting group;

$R^2$ is H or protected hydroxy;

$R^3$ and $R^4$ are independently H or protected hydroxy, or $R^3$ and $R^4$ taken together represent a protected keto group; and

$R^5$ is $C_{1-5}$alkyl, $C_{3-6}$cycloalkyl, $C_{6-10}$aryl, or $C_{6-10}$aryl substituted with one or more groups selected from $C_{1-5}$alkyl and $C_{1-5}$alkoxy, which comprises the steps of:

a) reacting a compound having the formula

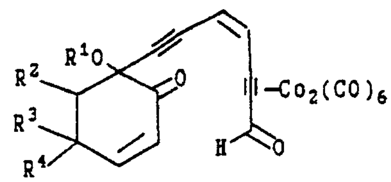

with a compound having the formula

$R^5$-S-A1($R^6$)$_2$

wherein $R^1$, $R^2$, $R^3$, $R^4$, and $R^5$ are as defined above and $R^6$ is $C_{1-5}$alkyl; and

b) reacting the product of step a with a reagent selected from Ti[O-($C_{1-5}$)alkyl]$_4$ and XTi[O-($C_{1-5}$)-alkyl]$_3$, wherein X is halogen.

**2.** A process for preparing a compound having the formula

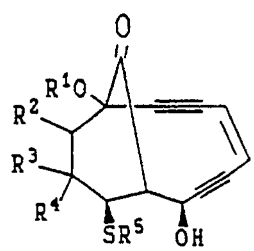

wherein

$R^1$ is hydroxy protecting group;

$R^2$ is H or protected hydroxy;

$R^3$ and $R^4$ are independently H or protected hydroxy, or $R^3$ and $R^4$ taken together represent a protected keto group; and

$R^5$ is $C_{1-5}$alkyl, $C_{3-6}$cycloalkyl, $C_{6-10}$aryl, or $C_{6-10}$aryl substituted with one or more groups selected from $C_{1-5}$alkyl and $C_{1-5}$alkoxy, which comprises the steps of:

a) reacting a compound having the formula

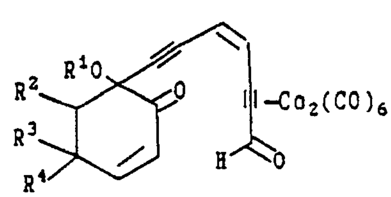

with a compound having the formula

$R^5$-S-A1($R^6$)$_2$

wherein $R^1$, $R^2$, $R^3$, $R^4$, and $R^5$ are as defined above and $R^6$ is $C_{1-5}$alkyl;
b) reacting the product of step a with a reagent selected from Ti[O-($C_{1-5}$)alkyl]$_4$ and XTi[O-($C_{1-5}$)-alkyl]$_3$, wherein X is halogen; and
c) removing the cobalt carbonyl group from the product formed in step b.

**3.** A process for preparing a compound having the formula

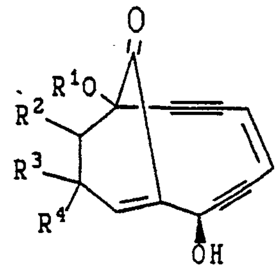

wherein

$R^1$ is hydroxy protecting group;

$R^2$ is H or protected hydroxy;

$R^3$ and $R^4$ are independently H or protected hydroxy, or $R^3$ and $R^4$ taken together represent a protected keto group; and

$R^5$ is $C_{1-5}$alkyl, $C_{3-6}$cycloalkyl, $C_{6-10}$aryl, or $C_{6-10}$aryl substituted with one or more groups selected from $C_{1-5}$alkyl and $C_{1-5}$alkoxy, which comprises the steps of:
a) reacting a compound having the formula

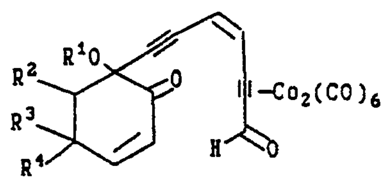

with a compound having the formula

$R^5$-S-A1($R^6$)$_2$

wherein $R^1$, $R^2$, $R^3$, $R^4$, and $R^5$ are as defined above and $R^6$ is $C_{1-5}$alkyl;
b) reacting the product of step a with a reagent selected from Ti[O-($C_{1-5}$)alkyl]$_4$ and XTi[O-($C_{1-5}$)-alkyl]$_3$, wherein X is halogen;
c) removing the cobalt carbonyl group from the product formed in step b; and
d) oxidizing the sulfide group of the product of step c into a sulfoxide to effect formation of the product.

**4.** A process for preparing a compound having the formula

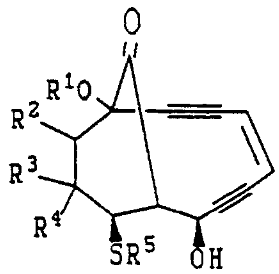

wherein

$R^1$ is hydroxy protecting group;

$R^2$ is H or protected hydroxy;

$R^3$ and $R^4$ are independently H or protected hydroxy, or $R^3$ and $R^4$ taken together represent a protected keto group; and

$R^5$ is $C_{1-5}$alkyl, $C_{3-6}$cycloalkyl, $C_{6-10}$aryl, or $C_{6-10}$aryl substituted with one or more groups selected from $C_{1-5}$alkyl and $C_{1-5}$alkoxy, which comprises the steps of:

a) reacting a compound having the formula

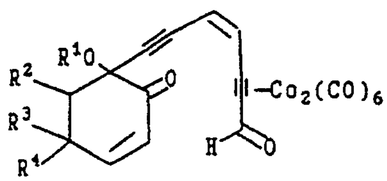

with a compound having the formula

$R^5$-S-A1$(R^6)_2$

wherein $R^1$, $R^2$, $R^3$, $R^4$, and $R^5$ are as defined above and $R^6$ is $C_{1-5}$alkyl;

b) reacting the product of step a with a reagent selected from Ti[O-$(C_{1-5})$alkyl$]_4$ and XTi[O-$(C_{1-5})$-alkyl$]_3$, wherein X is halogen; and

c) treating the product of step b with a peracid.

**5.** A compound having the formula

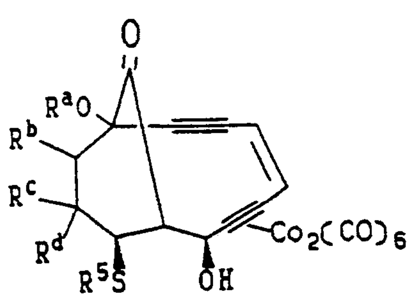

VIb

wherein

$R^a$ is hydrogen or a hydroxy protecting group;

$R^b$ is hydrogen, hydroxy, or a protected hydroxy;

$R^c$ and $R^d$ are independently hydrogen, hydroxy, or protected hydroxy, or $R^c$ and $R^d$ taken together represent an oxo group or a protected keto group; and

$R^5$ is $C_{1-5}$alkyl, $C_{3-6}$cycloalkyl, $C_{6-10}$aryl, or $C_{6-10}$aryl substituted with one or more groups selected from $C_{1-5}$alkyl and $C_{1-5}$alkoxy.

**6.** A compound of Claim 5 wherein $R^b$, $R^c$, and $R^d$ are each hydrogen.

**7.** A compound of Claim 5 wherein $R^c$, $R^b$, and $R^d$ are each hydrogen, $R^a$ is trialkylsilyl, and $R^5$ is phenyl.

**8.** A compound of Claim 5 wherein $R^b$, $R^c$, and $R^d$ are each hydrogen, $R^a$ is t-butyldimethylsilyl, and $R^5$ is phenyl.

**9.** A compound having the formula

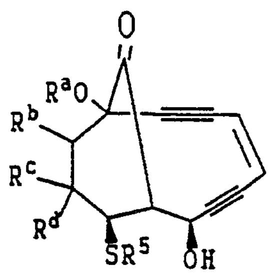

wherein

$R^a$ is hydrogen or a hydroxy protecting group;

$R^b$ is hydrogen, hydroxy, or a protected hydroxy;

$R^c$ and $R^d$ are independently hydrogen, hydroxy, or protected hydroxy, or $R^c$ and $R^d$ taken together represent an oxo group or a protected keto group; and

$R^5$ is $C_{1-5}$alkyl, $C_{3-6}$cycloalkyl, $C_{6-10}$aryl, or $C_{6-10}$aryl substituted with one or more groups selected from $C_{1-5}$alkyl and $C_{1-5}$alkoxy.

**10.** A compound of Claim 9 wherein $R^b$, $R^c$, and $R^d$ are each hydrogen.

**11.** A compound of Claim 9 wherein $R^b$, $R^c$, and $R^d$ are each hydrogen, $R^a$ is trialkylsilyl, and $R^5$ is phenyl.

**12.** A compound of Claim 9 wherein $R^b$, $R^c$, and $R^d$ are each hydrogen, $R^a$ is t-butyldimethylsilyl, and $R^5$ is phenyl.

**13.** A compound having the formula

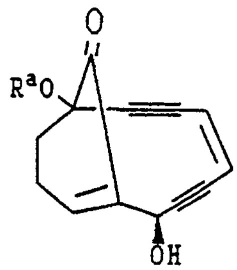

wherein $R^a$ is hydrogen or a hydroxy protecting group.

**14.** A compound of Claim 13 wherein $R^a$ is hydrogen.

**15.** A compound of Claim 13 wherein $R^a$ is t-butyldimethylsilyl.

**16.** The use of at least one compound of Claim 9 and/or Claim 14 for preparing a pharmaceutical composition for treating a malignant tumor.

**17.** A pharmaceutical composition comprising at least one compound of Claim 9 and/or Claim 14 and a pharmaceutically acceptable carrier.

**18.** A process for preparing the pharmaceutical composition of Claim 17 which comprises incorporating at least one compound of Claim 9 and/or Claim 14 into a pharmaceutically acceptable carrier.

European Patent Office

# EUROPEAN SEARCH REPORT

Application Number

**EP 91 10 6789**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A,D | JOURNAL OF ORGANIC CHEMISTRY. vol. 55, no. 6, 16 March 1990, EASTON US pages 1709 - 1711; PHILIP MAGNUS ET AL.: "STUDIES ON THE SYNTHESIS OF THE ANTITUMOR AGENTS ESPERAMICIN A1/CALICHEAMICIN GAMMA 1: STEREOSPECIFIC SYNTHESIS ...." * the whole document * | 3,13-18 | C 07 C 49/733<br>C 07 F 15/06<br>C 07 C 321/20<br>C 07 F 7/18<br>A 61 K 31/12 |
| A | TETRAHEDRON LETTERS. vol. 30, no. 15, March 1989, OXFORD GB pages 1905 - 1906; PHILIP MAGNUS ET AL.: "SYNTHETIC STUDIES ON THE ESPERAMICIN/CALICHEAMICIN ANTITUMOR ANTIBIOTICS. CONJUGATE ADDITION OF THIOL TO INITIATE 1,4-DIYL FORMATION" * the whole document * | 9,16-18 | |
| A,D | JOURNAL OF THE AMERICAN CHEMICAL SOCIETY. vol. 110, no. 20, 28 September 1988, GASTON, PA US pages 6921 - 6923; PHILIP MAGNUS ET AL.: "SYNTHESIS OF A REMARKABLY STABLE BICYCLO[7.3.1]DIYNENE ESPERAMICIN A1/CALICHEAMICIN GAMMA SYSTEM. STRUCTURAL REQUIREMENTS FOR FACILE FORMATION OF A 1,4-DIYL" * the whole document * | 1,16 | **TECHNICAL FIELDS SEARCHED (Int. Cl.5)** |
| A,D | TETRAHEDRON LETTERS. vol. 30, no. 7, February 1989, OXFORD GB pages 851 - 854; KIYOSHI TOMIOKA ET AL.: "SYNTHESIS OF THE CORE ENEDIYNE STRUCTURE OF ESPERAMICIN-CALICHEMICIN CLASS OF ANTITUMOR ANTIBIOTICS" * page 853 * | 3 | C 07 C<br>C 07 F<br>A 61 K |
| A,D | TETRAHEDRON LETTERS. vol. 30, no. 28, July 1989, OXFORD GB pages 3637 - 3640; PHILIP MAGNUS ET AL.: "SYNTHETIC STUDIES ON THE ESPERAMICIN/CALICHEAMICIN ANTITUMOR ANTIBIOTICS.SELENIUM DIOXIDE OXIDATION OF A BRIDGEHEAD TRIALKYLSILYL ENOL ETHER" * page 3638 * | 3,16 | |

-/-

The present search report has been drawn up for all claims

| Place of search | Date of completion of search | Examiner |
|---|---|---|
| Berlin | 16 July 91 | RUFET J.M.A. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

European Patent Office

# EUROPEAN SEARCH REPORT

Application Number

EP 91 10 6789

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A,D | TETRAHEDRON LETTERS. vol. 29, no. 34, August 1988, OXFORD GB pages 4217 - 4220; ANDREW S. KENDE ET AL.: "SYNTHESIS OF A CALICHEAMICIN DEOXYAGLYCONE MODEL BY AN INTRAMOLECULAR ACETYLIDE CYCLIZATION " * the whole document * ----- | 1 | |
| | | | TECHNICAL FIELDS SEARCHED (Int. Cl.5) |

The present search report has been drawn up for all claims

| Place of search | Date of completion of search | Examiner |
|---|---|---|
| Berlin | 16 July 91 | RUFET J.M.A. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention
E: earlier patent document, but published on, or after the filing date
D: document cited in the application
L: document cited for other reasons
&: member of the same patent family, corresponding document